# EUROPÄISCHE PATENTSCHRIFT

(11) **EP 1 819 317 B1**
(45) Veröffentlichungstag und Bekanntmachung des Hinweises auf die Patenterteilung: **07.05.2014**
(21) Anmeldenummer: 05802357.3
(22) Anmeldetag: 11.10.2005
(51) Int. Cl.: A61M 35/00

(54) **ARZNEIMITTEL UND SYSTEM ZUR PERKUTANEN ARZNEISTOFFVERABREICHUNG**
MEDICAMENT AND SYSTEM FOR THE PERCUTANEOUS ADMINISTRATION OF MEDICAMENTS
MEDICAMENT ET SYSTEME D'ADMINISTRATION PERCUTANEE DE SUBSTANCES PHARMACEUTIQUES

(30) Priorität: 12.10.2004 DE 102004049574
(43) Veröffentlichungstag der Anmeldung: 22.08.2007
(73) Patentinhaber: MedDrop Technology AG, 8512 Thundorf (CH); Barnikol-Keuten, Doris, 55128 Mainz (DE)
(72) Erfinder: BARNIKOL-KEUTEN, Doris, 55268 Nieder-Olm (DE); GULIK, Dieter, 21465 Reinbek-Ohe (DE)
(74) Vertreter: Müller, Claudia
(86) Internationale Anmeldenummer: PCT/EP2005/010909
(87) Internationale Veröffentlichungsnummer: WO 2006/040119

(56) Entgegenhaltungen:
- EP-A- 0 753 311
- WO-A-02/05754
- WO-A-92/18147
- FR-A- 2 641 463
- US-A- 5 244 677
- HINZ J ET AL: "BEHANDLUNG CHRONISCH THERAPIERESISTENTER WUNDEN MIT DEM BIOKATALYTISCH AKTIVIERBAREN SAUERSTOFFTRAEGER TETRACHLORDECAOXID" DIE MEDIZINISCHE WELT, Bd. 36, Nr. 8, 1985, Seiten 210-215, XP001152691 ISSN: 0025-8512
- JONSSON K ET AL: "TISSUE OXYGENATION, ANEMIA AND PERFUSION IN RELATION TO WOUND HEALING IN SURGICAL PATIENTS" ANNALS OF SURGERY, J. B. LIPPINCOTT COMPANY, PHILADELPHIA, US, Bd. 214, Nr. 5, 1991, Seiten 605-613, XP009012115 ISSN: 0003-4932
- SCHMALFUSS U ET AL: "MODIFICATION OF DRUG PENETRATION INTO HUMAN SKIN USING MICROEMULSIONS" JOURNAL OF CONTROLLED RELEASE, ELSEVIER, AMSTERDAM, NL, Bd. 46, Nr. 3, 2. Juni 1997 (1997-06-02), Seiten 279-285, XP000682261 ISSN: 0168-3659
- DELGADO-CHARRO M B ET AL: "DELIVERY OF A HYDROPHILIC SOLUTE THROUGH THE SKIN FROM NOVEL MICROEMULSION SYSTEMS" EUROPEAN JOURNAL OF PHARMACEUTICS AND BIOPHARMACEUTICS, ELSEVIER SCIENCE PUBLISHERS B.V., AMSTERDAM, NL, Bd. 43, Nr. 1, Januar 1997 (1997-01), Seiten 37-42, XP000678671 ISSN: 0939-6411

## Beschreibung

### Gegenstand der Erfindung

Die vorliegende Erfindung betrifft eine nach Zerstäuben mit einem Treibgas mit Sauerstoff angereichert vorliegende Mikroemulsion, enthaltend mindestens einen Arzneistoff zur perkutanen Verabreichung, die dadurch gekennzeichnet ist, dass die Sauerstoffanreicherung durch Zerstäuben mit einem sauerstoffhaltigen Treibgas oder durch Zerstäuben mit einem Treibgas einer Mikroemulsion, enthaltend einen Zusatz zur Verbesserung der Sauerstoffversorgung, vorliegt.

### Hintergrund

Bei Verabreichung von Arzneistoffen als Wirkstoffsubstanzen an einen Patienten ist bei der Dosierung immer zwischen erwünschter Wirkung und unerwünschten Nebenwirkungen auf den Organismus abzuwägen. Daher ist es wünschenswert, den Arzneistoff möglichst direkt an den Wirkort zu bringen, um somit mit minimalen Gesamtdosierungen arbeiten zu können und den Organismus des Patienten möglichst wenig zu belasten, und dennoch am Wirkort den nötigen Wirkspiegel zu erreichen. Dies kann durch perkutane Verabreichung von Arzneistoffen erreicht werden.

Die Haut, insbesondere die obere Hornschicht, stellt allerdings, eine nur schwer zu überwindende Barriere dar. Dies gilt im Besonderen für wasserlösliche oder schwer lösliche Arzneistoffe.

Ein herkömmliches Verfahren zur perkutanen Verabreichung von Arzneistoffen ist das Auftragen von Salben, Cremes oder Gelen auf die Haut. Um die Permeation der Wirksubstanzen zu verbessern, verwendet man sogenannte Penetrationsverstärker, wie Sulfoxide, Alkohole, Fettsäuren, Anoide, Fuside u.v.m. Diese Substanzen verringern den Penetrationswiderstand der Hornschicht und erleichtern die Permeation der Arzneistoffe.

Nachteile dieses Verfahrens sind nur ungenaue Dosierungsmöglichkeiten.

Dadurch muss der Gehalt an Arzneistoffen in den Zubereitungen vorsichtshalber gering gehalten werden, wodurch auch in den Zielgeweben nicht der gewünschte, hohe Wirk-Spiegel erreicht wird. Zudem ist trotz Verwendung der Penetrationsverstärker die Eindringtiefe der klassischen Zubereitungen nur sehr gering. Des Weiteren sind unterschiedliche Verfahrensweisen bekannt, die Barriere der Haut zu überwinden, (yergl. Müller/Hildebrand; Pharmazeutische Technologie: Moderne Arzneiformen, ISBN 3-8047-1549-4, Kapitel 13).

Insbesondere hat man transdermale, therapeutische Systeme (TTS) entwickelt. TTS sind technische Einrichtungen, die auf eine bestimmte Hautstelle haftend aufgesetzt werden und eine bestimmte Dosis des Arzneistoffs nach verschiedenen Mechanismen mit einem bestimmten zeitlichen Verlauf durch die Haut an den Organismus diffusiv abgeben. Zielsetzung ist hierbei insbesondere eine systemische Wirkung mit einem definierten Profil des Wirkspiegels. Um die Permeation des Arzneistoffes in die Haut zu beschleunigen, besitzen die TTS-Systeme auch Ultraschall-Köpfe oder Elektroden, um Stromstöße an die Haut abzugeben und somit durch mechanische oder elektrische Reize eine
Porenbildung in der Haut zu fördern.

Nachteilig ist hier, dass eine gezielte, lokale Anwendung mittels TTS nicht möglich ist. Hinzu kommt, dass nicht alle Arzneistoffe diffusiv verabreicht werden können. Dies gilt vor allem für wasserlösliche und schwer lösliche Arzneistoffe.

Weiter werden Arzneistoffe in Mikroemulsionen auf die Haut aufgebracht. Aufgrund der geringen Grenzflächenspannung und großer Grenzfläche innerhalb der Mikoemulsion können wasserlösliche, fettlösliche und schwer lösliche Arzneistoffe darin dispergiert werden. Mit Hilfe einer Mikroemulsion gelingt es innerhalb kurzer Zeit die Arzneistoffe in die Hornschicht der Haut (Stratum Corneum) einzubringen. Allerdings gelingt es auch mit Hilfe von Mikroemulsionen allein nicht zufriedenstellend, die Barrierefunktion der Haut in gewünschtem Grad vorübergehend aufzuheben und alle Arten von Arzneistoffen durch die Haut zu applizieren.

Die US 5,244,677 A betrifft Auflagen bzw. Pflaster zur transdermalen Verabreichung von Medikamenten. Es werden jedoch weder spezielle Arzneistoffe noch Zusätze zur Anreicherung mit Sauerstoff offenbart.

J. Hinz et al. berichten in ,Die Medizinische Welt, Band 36, Nr.8,1985,Seiten 210-215' über die Behandlung chronischer Wunden mit Tetrachlordecaoxid in wässriger Lö-sung.

K. Jonsson et al. beschreiben in ,Annals of Surgery, J.B. Lippincott Company, Philadelphia, US, Bd. 214, Nr.5, 1991, Seiten 605-613, XP009012115 ISSN:0003-4932' den Verlauf einer Wundheilung in Gegenwart von vorhandenem Sauerstoff und der damit verbundenen Kollagenablagerung.

In der WO 02/05754 A werden W/O-Emulsionen zum Einreiben oder Aufsprühen beschrieben, welche künstliche Sauerstoffträger enthalten können, um Haut und Haare von außen mit notwendigen Nährstoffen, auch Sauerstoff zu versorgen.

Aus der FR 2 641 463 A sind Gele, Lotionen, Cremes und Shampoos bekannt, welche Hämocyanin zur verbesserten Versorgung der Haut mit Sauerstoff aufweisen.

In den Druckschriften EP 0 753 311 A1, WO 92/181147, werden Mikroemulsionen offenbart, welche wirkstoffspezifische Penetrationsverstärker wie Cholesterol für DPH (WO92/181147) enthalten.

U. Schmalfuss et al, Journal of Controlled Release, Elsevier, Amsterdam, NL, Bd. 46, Nr. 3, 2. Juni 1997, sowie M. B. Delgado-Charro et al., European Journal of Pharmaceutics and Biopharmaceutics, Elsevier Science Publisher B.V., Amsterdam, NL, Bd. 43, Nr.1, 1. Januar 1997-01, Seite 37-42, XP000678671 ISSN:0939-6411, berichten über den Wirkstofftransport durch Mikroemulsionen.

Aufgabe der Erfindung ist es, die oben genannten Nachteile des Stands der Technik zu beheben.

Speziell ist es Aufgabe der Erfindung, Zubereitungen (im Folgenden Arzneimittel genannt) zur Verfügung zu stellen, die in zufriedenstellender Weise die Barriere der Haut durchdringen.

Weiter ist es Aufgabe der Erfindung, ein System zur Verfügung zu stellen, mit dem es möglich ist, an jeder Hautstelle die Barriere der Haut zu durchdringen und eine Wirksubstanz oder eine Kombination von Wirksubstanzen perkutan zu applizieren. Weiter ist es eine Aufgabe der Erfindung, ein System zur Verfügung zu stellen, mit dem die zu applizierenden Arzneistoffe genau dosiert werden können.

Eine weitere Aufgabe der Erfindung ist es, die maximale Tagesdosis lokal zu applizieren.

Überraschenderweise wurde gefunden, dass diese und weitere, nicht genannte Aufgaben mit Hilfe eines erfindungsgemäßen Systems zur perkutanen Verabreichung von Arzneistoffen, aufweisend eine Mikroemulsion, in der die Arzneistoffe eingebracht sind, und einer Vorrichtung zum Zerstäuben der Mikroemulsion, vorzugsweise in sauerstoffhaltiger Atmosphäre, gelöst werden (Unter Zerstäuben wird hier die Feindispergierung einer Flüssigkeit mit Hilfe eines Treibgases verstanden).

Weiterhin gelöst werden diese Aufgaben durch eine mit Sauerstoff angereicherte Mikroemulsion, enthaltend mindestens einen Arzneistoff zur perkutanen Verabreichung, wobei die Sauerstoffanreicherung durch Zerstäuben mit einem sauerstoffhaltigen Treibgas oder durch Zerstäuben mit einem Treibgas einer Mikroemulsion, enthaltend einen Zusatz zur Verbesserung der Sauerstoffversorgung, vorliegt. Als Treibgas kann Inertgas, Luft oder Treibgas mit einer Sauerhaltigen Atmosphäre von größer 25 Vol.% des Treibgases gewählt werden.

Als Zusätze zur verbesserten Sauerstoffversorgung der Haut in der Arzneistoffe enthaltenden Mikroemulsion zur perkutanen Verabreichung können Hämoglobin oder Myoglobin gewählt werden.

Die Kombination der verschiedenen Mechanismen des neuen Verfahrens kann zu erheblichen Synergie-Effekten bei der Permeation von Wirksubstanzen in die Haut führen, wie nachstehend erläutert ist.

Insbesondere eignen sich erfindungsgemäße Mikroemulsionen zur Herstellung eines zerstäubbaren Mittels zur Behandlung rheumatischer Beschwerden, des peripheren, komplexen Schmerzsyndroms, von Wunden, Prellungen, Zerrungen, Sportverletzungen, Neuralgien, viralen Infektionen, Hämatomen oder degenerierter Haut.

Durch die außerordentlich kleinen Tröpfchen des Hochleistungszerstäubers wird die mit Wirksubstanz beladene Mikro-Emulsion fein verteilt auf die Haut aufgebracht. Wegen der geringen Oberflächenspannung der Mikro-Emulsion ergibt sich hierbei ein enormer Spreiteffekt. Die Hornschicht der Haut und die Mikro-Emulsion besitzen ähnliche Überstrukturen, wie Lamellen oder Tubuli, aufgebaut aus Bilipidschichten. Diese Überstrukturen der Hornschicht tragen entscheidend mit zum Permeationswiderstand dieser Schicht bei. Das feindisperse Aufbringen der Tröpfchen führt vermutlich zum "Verschmelzen" der MikroEmulsion mit der Hornschicht gemäß dem Prinzip "Similia similibus". Durch das Verschmelzen lösen sich die genannten Überstrukturen auf und die Wirksubstanzen können verstärkt in die Haut eindiffundieren. Die Verwendung von Sauerstoff als Treibgas bewirkt die Anreicherung von Sauerstoff in den lipidhaltigen Tröpfchen des Zerstäubers. Dieser Sauerstoff wird - wie die Wirksubstanzen - mit in die Hautschicht eingetragen, was zu einer Steigerung des Sauerstoffpartialdrucks in der Haut führt. Dieser erhöhte Partialdruck regt den mikrozirkulatorischen Fluss stark an. Hierdurch werden die eindiffundierten Wirkstoffsubstanzen verstärkt konvektiv nach innen ins Gewebe mitgenommen.

Die kombinierte Verwendung von Mikro-Emulsionen, feinen Tröpfchen und Sauerstoff in dem erfindungsgemäßen Verfahren bewirkt also eine Steigerung der Permeation von Wirksubstanzen in drei hintereinander geschalteten Stufen:
1. Es kommt zu einer sehr feinen Verteilung und Spreitung der MikroEmulsion und damit der Wirksubstanzen auf der Hautoberfläche.
2. Die Hornschichtbarriere wird überwunden und
3. es verstärkt sich der mikrozirkulatorische Transport durch die Haut, nämlich erstens durch die Hochleistungszerstäubung, zweitens durch die Mikro-Emulsion und drittens durch den Sauerstoff.

### Detaillierte Beschreibung der Erfindung

Die Haut ist das größte Organ des Körpers, welches den Abschluss nach außen bildet. Sie muss in ihrer Funktion eine Reihe von Leistungen erbringen.

Die Schutzfunktion gegen mechanische Einwirkungen wie Stöße, Druck, Reibung, und gegen das Eindringen von Bakterien, Viren und Pilzen durch einen Säuremantel steht an erster Stelle. Weiterhin schützt die Haut gegen Hitze, Kälte, Licht und schädliche Stoffe.

Die Haut ist auch Sinnesorgan, spezielle Sensoren nehmen Druck, Temperatur, Schmerz und Juckreiz wahr.

Die Haut greift auch durch Regulation des Wasser- und Wärmehaushaltes regulierend in die Funktion des Gesamtorganismus ein.

Grob gesehen besteht die Haut aus drei Schichten, aus der Subcutis (Unterhaut), aus dem Corium (Lederhaut) sowie aus der Epidermis (Oberhaut).

Die Unterhaut besteht aus Fett, großen Blutgefäßen, Drüsen sowie kleinen Muskeln. Sie dient z.B. als "Vorratskammer" sowie der Dämpfung mechanischer Einwirkungen. Die Lederhaut bedingt mit ihren Kollagen- und Elastinfasera den Halt und Elastizität der Haut, damit auch die Reißfestigkeit. In der Lederhaut befinden sich auch die Sinneszellen (Sensoren) zur Aufnahme der genannten Empfindungen. Sie enthält viel Hyaluronsäure und Chondroitinsulfat - also Glukosamino-Glucane - welche als reversible Gele den Transport von biologischen Molekülen und Zellbewegungen ermöglichen.

Für den Abschluss des Organismus nach außen ist die Epidermis von besonderer Bedeutung und besonderem Interesse, da diese Schicht die Integrität der Haut insgesamt gewährleistet, wobei die alleräußerste Schicht, die Homschicht, eine entscheidende Rolle spielt.

Diese Schicht besteht aus etwa 10 Zeil-Lagen verhornender, d.h. abgestorbener, flacher Zellen (Hornzellen, Stratum corneum); sie teilt man noch weiter auf in eine obere lockere Schicht (Stratum disjunctum) und in eine untere festere Schicht, das Stratum conjunctum. Die Hornzellen schilfern ständig nach außen ab und entstehen durch Teilung im darunter befindlichen sogenannten Stratum germinativum, der Keimschicht.

Die besondere Mikrostruktur des Stratum corneum besteht in flachen, Ziegelsteinähnlich verhornenden Zellen (Corneozyten). Besonders strukturiert ist die interzelluläre Matrix. Sie besteht aus, ungefähr zur Hautoberfläche parallelen, Lipoid-Doppelschichten: Im Stratum corneum wechseln sich etwa einhundert Hydro- und Lipophasen ab. Galenisch gesehen stellt die Hornschicht eine sogenannte Wasser in Öl -Emusion in Form einer lamellaren Doppelschicht dar. Diese, nur etwa 12 µm dünne, sich ständig regenerierende Schicht bildet mit Hilfe ihrer komplexen biphasigen Überstrukturen den sicheren Schutz für die darunter befindlichen Zellen des Stratum germinativum: Ohne die Hornschicht besteht ein "Wundbett".

Die Hornschicht der Haut ist für den Abschluss nach außen von besonderer Bedeutung, vor allem in ihrer Barrieren-Funktion. Dies trifft bezüglich der Dichte, des Sauerstoffpartialdrucks (PO₂), des pH- Wertes und in Bezug auf den Wassergehalt zu.

Besonders wichtig ist die Barriere für Wasserstoff-Ionen, die einen Säure-Schutzmantel bilden. Gleichermaßen wichtig ist die Barriere für Sauerstoff, indem sie diesem einen großen Diffusionswiderstand entgegensetzt. Dies führt zu einer Erniedrigung des Sauerstoffpartialdruckes der Luft von 150 Torr auf etwa 50 Torr. Somit werden die vitalen Zellen des Hautepithels der intakten Haut vor einem zu hohen, oxidativ-schädlichen Sauerstoff-Partialdruck geschützt.

So vorteilhaft die effektive Barrierefunktion in der Hornschicht für den Organismus ist, so nachteilig erweist sie sich für einen transdermalen Transport von Arzneistoffen. In solchen Fällen muss die korneale Barriere vorübergehend aufgehoben werden.

Überraschenderweise wurde gefunden, dass die Barrierefunktion der Haut durch Einbringen von Sauerstoff in die Hornschicht und somit die Erhöhung des Sauerstoffpartialdrucks auf der Gewebeseite des Stratum corneums zu einem verbesserten transdermalen Transport von Arzneistoffen führt.

Durch die Erhöhung des Sauerstoffpartialdrucks auf der Gewebeseite des Stratum corneums erhöht sich der Transmembrandruck des Sauerstoffs, der vermutlich eine Ursache für den verbesserten transdermalen Transport von Arzneistoffen ist.

Aufgrund der oben beschriebenen lamellaren Struktur aus wechselnden Wasser-und Ölphasen des Stratum corneum sind Mikroemulsionen besonders geeignet in das Stratum Corneum einzudringen (vergleiche Müller/Hildebrand;
Pharmazeutische Technologie: Moderne Arzneiformen, ISBN 3-8047-1549-4, Kapitel 15). Diese werden in einer bevorzugten Ausführungsform der Erfindung als Vehikelsysteme für Sauerstoff oder Arzneistoffe sowie Basisstoffe für Arzneimittel verwendet.

Solche Mikroemulsionen sind bekannt und werden in der Kosmetik und Arzneimittelindustrie verwendet. Diese sind beispielsweise unter dem Handelsnamen "Nanoemulsion" der Firma Sangui AG kommerziell erhältlich.

Mikroemulsionen im Sinne der Erfindung sind thermodynamisch stabile Systeme, die zumindest Wasser, Tenside und Lipid aufweisen. Unter einem Tensid versteht man Emulgatoren, die ionisch oder nicht ionisch sein können. Beispiele für Tenside, die verwendet werden können, sind unter dem Handelsnamen Tween, Span und Synperonic PEL 101 bekannt.

Lipide, die verwendet werden können sind fette Öle oder Mineralöle, beispielsweise Isopropylmyristat und Isopropylpalmiat.

Mikroemulsionen, die im Rahmen dieser Erfindung verwendet werden können, können Öl in Wasser Mikroemulsionen oder Wasser in Öl Mikroemulsionen sein. Dabei bilden sich Öltröpfchen in einer Wassermatrix bzw. Wassertröpfchen in einer Ölmatrix.

Solche Mikroemulsionen weisen Tröpfchengrößen im Bereich von 10 nm bis 1 µm, vorzugsweise von 10 nm bis 500 nm, besonders bevorzugt von 10 nm bis 300 nm auf.

Die mittlere Tröpfchengröße einer Mikroemulsion, die im Rahmen der Erfindung verwendet werden kann ist nicht beschränkt. Vorzugsweise ist die mittlere Tröpfchengröße kleiner 300 nm, besonders bevorzugt kleiner 150 nm.

Solche Mikroemulsionen weisen bevorzugt Grenzflächen von mehr als 200 m² pro mL, besonders bevorzugt von mehr als 400 m² pro mL und ganz besonders bevorzugt von mehr als 600 m pro mL auf.

Aufgrund des hydrophilen und lipophilen Anteils der Mikroemulsionen sowie der geringen Grenzflächenspannung und der großen Grenzfläche ist es möglich in Mikroemulsionen sowohl wasserlösliche als auch Fettlösliche und / oder schwer lösliche Arzneistoffe zu dispergieren. Je nach Wirksubstanz und gewünschter Wirkung wird dabei die Wahl der Tenside getroffen. Ionische Tenside sind im

Allgemeinen besonders effektiv, während nichtionische Tenside besonders hautschonend sind.

Erfindungsgemäße Mikroemulsionen betreffen unter anderem die medizinische Anwendung von flüssigen Arzneimitteln auf Basis von Mikroemulsionen in der Schmerztherapie, zur Behandlung von Durchblutungsstörungen, und zur Wundheilung degenerierter Haut, z.B. bei älteren Menschen.

Die Mikroemulsionen können neben den Grundstoffen der Mikroemulsion Basisstoffe für Arzneimittel und Arzneistoffe aufweisen. Diese Basisstoffe und Arzneistoffe können natürlichen und synthetischen Ursprungs sein. Im Rahmen dieser Erfindung sind Basisstoffe und Arzneistoffe auf natürlichen Ursprungs besonders bevorzugt, ohne dass dies beschränkend sein soll.

Insbesondere weisen erfindungsgemäße Mikroemulsionen Arzneistoffe auf, die in den Ansprüchen 4-8 beschrieben und ausgewählt sind:
aus der Gruppe, umfassend analgetisch, anästhesierend, anti-phlogistisch, spasmolytisch, hyperämisierend wirkende Arzneistoffen; oder:
aus der Gruppe, umfassend antibakteriell, antikoagulierend, aquaretisch, antiviral, antioxidativ, epithelisierend, Keratolytisch, hautnährend, wundheilend, antimykotisch wirkende Arzneistoffe, oder.
aus der Gruppe, umfassend antiallergisch, ödemprotektiv, antimykotisch, hautregenerierend, brennlindernd, hämolytisch, mitosehemmend, bindegewebsregenerierend, antispastisch wirkende Arzneistoffe; oder.
aus der Gruppe, umfassend antimikrobiell, immunmodulatonsch, juckreizstillend, adstringierend, lokal-anästhesierend wirkende Arzneimittel.

Insbesondere können in erfindungsgemäßen Mikroemulsionen auch Kombinationen aus Arzneistoffen vorliegen.

Beispiele für natürliche Basisstoffe sowie deren Wirkung sind in Tabelle 1 dargelegt. Basisstoffe, die im Rahmen dieser Erfindung verwendet werden können, sind jedoch nicht darauf beschränkt.

**Tabelle 1**

| Natürliche Basisstoffe sowie deren Wirkung | |
|---|---|
| **Basisstoffe** | **Wirkung** |
| Aloe vera | • durchblutungsfördernd |
| | • feuchtigkeitsspendend |
| | • entzündungshemmend |
| | • hautstraffend |
| | • hautnährend |
| Amicaöl (fett) | • schmerzstillend |
| | • entzündungshemmend |
| | • hyperämisierend |
| | • durchblutungsfördernd |
| | • wundheilend |
| Avocadoöl | • feuchtigkeitsbindend |
| | • regenerierend |
| | • juckreizstillend |
| | • wundheilend |
| | • hautnährend |
| Borretschsamenöl | • hautregenerierend |
| | • juckreizstillend |
| Centellaöl | • regenerierend |
| | • bindegewebs regulierend (Narben) |
| | • antiphlogistisch |
| | • wundheilend |
| Johanniskrautöl | • antiphlogistisch |
| | • analgetisch |
| | • hyperämisierend |
| | • antispasmodisch |
| Jojobaöl | • entzündungshemmend |
| | • regenerierend |
| | • wundheilend |
| Maiskeimöl | • antioxidativ |
| Mandelöl | • regenerierend |
| | • nährend |
| Nachtkerzenöl | • wundheilend |
| | • antibakteriell |
| | • juckreizstillend |
| Neemöl | • antibakteriell |
| | • antimykotisch |
| Olivenöl | • hyperämisierend |
| | • durchblutungsfördernd |
| | • wundheilend |
| Ringelblumenöl | • antiphlogistisch |
| | • antirheumatisch |
| | • durchblutungsfördernd |
| Sheabutter | • wundheilend |
| | • regenerierend |
| Traubenkernöl | • adstringierend |
| Weizenkeimöl | • regenerierend |
| | • hautnährend |
| Wildrosenöl Hagebuttensamenöl | • feuchtigkeitsspendend |
| | • hautregenernierend |
| | • juckreizstillend |
| | • nährend |
| | • wundheilend |

Die Arzneistoffe, die im Rahmen dieser Erfindung verwendet werden können sind nicht beschränkt. Dabei können natürliche und synthetische Arzneistoffe verwendet werden. Im Rahmen dieser Erfindung sind natürliche Arzneistoffe, gewonnen aus Pflanzen, bevorzugt. Besonders bevorzugt als Arzneistoffe sind ätherische Öle, die aus Pflanzenteilen gewonnen werden können. Beispiele für Pflanzenarten und Gattungen, einschließlich ihrer Chemotypen, die in den unterschiedlichsten Pflanzenteilen ätherische Öle enthalten, die in Mikroemulsionen im Rahmen dieser Erfindung als Arzneistoffe verwendet werden können, sowie deren therapeutische Wirkung bei äußerlicher Anwendung sind in Tabelle 2 dargelegt, diese sind jedoch nicht darauf beschränkt.

**Tabelle 2**

| Pflanzenarten und Gattungen einschließlich ihrer Chemotypen, die in den unterschiedlichsten Pflanzenteilen ätherische Öle enthalten, sowie deren therapeutische Wirkung bei äußerlicher Anwendung | | |
|---|---|---|
| **Name** | **Arten** / **Gattung** / **Chemotypen** | **Eigenschaften** |
| Angelikaöl | Angelica | • hautregenerierend |
| Baldrianöl | Valeriana | • aquaretisch |
| | | • hautregenerierend |
| Basilikumöl | Ocimum Chemotyp Methylchavicol | • antibakteriell |
| | | • antispasmodisch |
| | | • antiviral |
| | | • antiphlogistisch |
| | | • analgetisch |
| | | • entstauend |
| | | • phlebotonisch |
| Baytil Pimentöl | Pimenta | • antibakteriell |
| | | • antimykotisch |
| | | • antiviral |
| Beifußöl | Artemisia | • antiviral |
| Benzoeharz | Styrax | • antiphlogistisch |
| | | • antiseptisch |
| | | • hautregenerierend |
| | | • zellerneuernd |
| Bergamotteöl | Citrus aurantium var. Bergamia | • antiseptisch |
| | | • epithelisierend |
| | | • wundheilend |
| | | • hautregenerierend |
| Bergbohnenkrautöl | Satureja montana | • analgetisch |
| | | • antibakteriell |
| | | • antimykotisch |
| | | • antiseptisch |
| | | • immunmodulatorisch |
| Birkenöl | Betula 98% Methylsalicylat | • antiphlogistisch |
| | | • antirheumatisch |
| | | • antispasmodisch |
| | | • schmerzstillend |
| | | • vasodilatatorisch |
| Cajeputöl | Melaleuca | • antibakteriell |
| | | • antiviral |
| | | • phlebotonisch |
| Cassiazimtöl | Cinnamomum cassia | • antibakteriell |
| | | • antikoagulierend |
| | | • antimykotisch |
| | | • antiviral |
| | | • hyperämisierend |
| Cistroseöl | Cistus | • antibakteriell |
| | | • antihämorrhagisch |
| | | • antiviral |
| Eukalyptusöl | Eucalyptus | • analgetisch |
| | | • antibakteriell |
| | | • antimykotisch |
| | | • antiphlogistisch |
| | | • antiviral |
| Fenchelöl | Foeniculum | • analgetisch |
| | | • entwässernd |
| Fichtennadelöl | Abies | • antiphlogistisch |
| | | • hyperämisierend |
| Galbanumöl | Ferula | • antiphlogistisch |
| | | • antiseptisch |
| | | • wundheilend |
| Geranienöl | Pelargonium graveolens | • adstringierend |
| | | • antibakteriell |
| | | • antimykotisch |
| | | • entstauend |
| | | • hautpflegend |
| | | • phlebotonisch |
| | | • wundheilend |
| Geranienöl "echte Geranie" | Geranium macrorrhizum | • antiseptisch |
| | | • epithelisierend |
| Gewürznelkenöl | Eugenia caryophyllata | • antibakteriell |
| | | • antimykotisch |
| | | • antiviral |
| Ho-Baumöl | Cinnamomum | • antibakteriell |
| | | • antimykotisch |
| | | • antiviral |
| Immortellenöl Strohblumenöl | Helichrysum | • analgetisch |
| | | • antikoagulierend |
| | | • epithelisierend |
| Ingweröl | Zingiber | • analgetisch |
| | | • hyperämisierend |
| Kamillenöl blau | Matricaria camomilla | • antiphlogistisch |
| | | • wundheilend |
| Kamillenöl römisch | Anthemis nobilis | • analgetisch |
| | | • antiphlogistisch |
| Kamillenöl wild | Ormensis mixtra | • antibakteriell |
| | | • antimykotisch |
| | | • wundheilend |
| Kampferöl | Cinnamomum | • anaesthetisch |
| | | • analgetisch |
| | | • antibakteriell |
| | | • antiinfektiös |
| | | • antimykotisch |
| | | • antirheumatisch |
| | | • antiviral |
| | | • aquaretisch |
| | | • hyperämisierend |
| | | • immunmodulatorisch |
| | | • rheumatische Schmerzen |
| | | • spasmolytisch |
| Kiefernöl | Pinus | • antibakteriell |
| | | • hyperämisierend |
| | | • ödemprotektiv |
| Latschenkiefernöl | Pinus mugo | • antiphlogistisch |
| | | • immunmodulatorisch |
| Lavendelöl | Lavendula | • analgetisch |
| | | • antibakteriell |
| | | • antikoagulierend |
| | | • antimykotisch |
| | | • antiphlogistisch |
| | | • epithelisierend |
| | | • juckreizstillend |
| Lavendelsalbeiöl | Salvia | • analgetisch |
| | | • antiinfektiös |
| | | • antispasmodisch |
| | | • tonisierend |
| Lemongrasöl | Cymbopogon | • antibakteriell |
| | | • antiphlogistisch |
| | | • antiviral |
| | | • gefäßerweiternd |
| | | • immunmodulatorisch |
| Lorbeeröl | Laurus | • analgetisch |
| | | • antibakteriell |
| | | • antikoagulierend |
| | | • antispasmodisch |
| | | • mukolytisch |
| | | • ödemprotektiv |
| Majoranöl | Origanum | • analgetisch |
| | | • antibakteriell |
| | | • antispasmodisch |
| | | • aquaretisch |
| Manukaöl | Leptospermum | • antibakteriell |
| | | • antimykotisch |
| | | • antiphlogistisch |
| | | • antirheumatisch |
| | | • beruhigend |
| | | • hautregenerierend |
| | | • juckreizstillend |
| Melissenöl | Melissa | • analgetisch |
| | | • antiviral |
| | | • entzündungshemmend |
| | | • immunmodulatorisch |
| | | • phlebotonisch |
| Myrrheöl | Commiphora | • antibakteriell |
| | | • antiphlogistisch |
| | | • antiviral |
| | | • epithelisierend |
| | | • hautregenerierend |
| Niaouliöl | Melaleuca | • analgetisch |
| | | • antiinfektiös |
| | | • antimykotisch |
| | | • antiviral |
| | | • immunmodulatorisch |
| | | • phlebotonisch |
| Oreganoöl | Origanum | • analgetisch |
| | | • antibakteriell |
| | | • antimykotisch |
| | | • antiviral |
| | | • hyperämisierend |
| | | • immunmodulierend |
| Patchouliöl | Pogostemon | • analgetisch |
| | | • antiinfektiös |
| | | • antimykotisch |
| | | • antiphlogistisch |
| | | • aquaretisch |
| | | • entstauend |
| | | • epithelisierend |
| | | • immunmodulatorisch |
| Petit Grainöl | Citrus aurantium | • antiinfektiös |
| | | • antiphlogistisch |
| | | • antispasmodisch |
| Peru-Balsam | Myroxylon | • antibakteriell |
| | | • antiphlogistisch |
| | | • antispasmodisch |
| Pfefferöl (schwarz) | Piper | • analgetisch |
| | | • antibakteriell |
| | | • antiviral |
| | | • aquaretisch |
| | | • hyperämisierend |
| Pfefferminzöl | Mentha | • analgetisch |
| | | • anästhetisierend |
| | | • antibakteriell |
| | | • antimykotisch |
| | | • antiparasitisch |
| | | • antiviral |
| | | • epithelisierend |
| | | • kühlend |
| | | • spasmolytisch |
| Pimentöl | Pimenta | • antibakteriell |
| | | • antimykotisch |
| | | • antiviral |
| Rainfarnöl | Tanacetum | • analgetisch |
| | | • antiallergisch |
| | | • juckreizstillend |
| | | • phlebotonisch |
| Ravensara | Ravensara | • antibakteriell |
| | | • antimykotisch |
| | | • antiviral |
| Rosenöl | Rosa damaszena | • antiphlogistisch |
| | | • antiviral |
| | | • hautregenerierend |
| Rosmarinöl | Rosmarinus | • analgetisch |
| | Chemotyp Cineol "marokkanisch" | • aquaretisch |
| | | • fungizid |
| | | • hyperämisierend |
| Sade-Baumöl | Juniperus | • analgetisch |
| | | • hyperämisierend |
| Salbeiöl | Salvia | • antibakteriell |
| | | • antimykotisch |
| | | • antiviral |
| Sandelholzöl | Santalum | • entstauend |
| | | • epithelisierend |
| Schafgarbeöl | Achillea | • analgetisch |
| | | • antiphlogistisch |
| | | • epithelisierend |
| Schwarzkümmelöl | Nigella | • analgetisch |
| | | • antiallergisch |
| | | • antiphlogistisch |
| Speiklavendelöl | Lavendula spica | • analgetisch |
| | | • antiinfektiös |
| | | • antiviral |
| | | • fungizid |
| Tagetesöl | Tagetes | • antimykotisch |
| Tea Treeöl | Melaleuca | • analgetisch |
| | | • antibakteriell |
| | | • antimykotisch |
| | | • antiparasitisch |
| | | • antiphlogistisch |
| | | • antiviral |
| | | • epithelisierend |
| | | • immunmodulatorisch |
| | | • phlebotonisch |
| Texas-Zederöl | Juniperus mexicana | • entstauend |
| | | • aquaretisch |
| Thujaöl | Thuja | • antiinfektiös |
| | | • antiviral |
| | | • aquaretisch |
| | | • epithelisierend |
| | | • wundheilend |
| Thymianöl | Thymus vulgaris | • antibakteriell antimykotisch |
| | Chemotyp Linalool und Geraniol | |
| | | • antiviral |
| | Thymus | • antibakteriell |
| | Chemotyp Thujanol | • antiviral |
| | | • immunmodulierend |
| | Thymus | • analgetisch |
| | Chemotyp Thymol und Carvacrol | • antiinfektiös |
| | | • immunmodulatorisch |
| Vetiveröl | Vetiveria | • hautpflegend |
| | | • hyperämisierend |
| Wacholderöl | Juniperus | • antibakteriell |
| | | • antirheumatisch |
| | | • aquaretisch |
| Weihrauchöl | Boswellia | • antiphlogistisch |
| | | • epithelisierend |
| | | • immunmodulierend |
| Weißtannenöl | Abies | • antiseptisch |
| | | • hyperämisierend |
| Wintergrünöl | Gaultheria | • antiphlogistisch |
| | | • antispasmodisch |
| | | • schmerzstillend |
| | | • vasodilatatorisch |
| Ysopöl | Hyssopus | • antibakteriell |
| | | • antiviral |
| | Hyssopus var. | • antiphlogistisch |
| | Decumbens | • antiviral |
| Zimtöl | Cinnamomum verum | • antibakteriell |
| | | • antimykotisch |
| | | • antiparasitisch |
| | | • antiviral |
| | | • hyperämisierend |
| | | • immunmodulatorisch |
| Zitronenöl | Citrus | • adstringierend |
| | | • antibakteriell |
| | | • antikoagulierend |
| | | • antiviral |
| | | • phlebotonsich |
| Zypressenöl | Cupressus | • adstringierend |
| | | • aquaretisch |
| | | • entstauend |
| | | • phlebotonisch |

In Tabelle 3 sind bevorzugt verwendete Arzneistoffe sowie deren Wirkeigenschaften aufgeführt. Diese sind unterteilt in ätherische Öle, Pflanzenextrakte und synthetische Monosubstanzen. Die im Rahmen dieser Erfindung einsetzbaren Arzneistoffe sollen allerdings nicht darauf beschränkt sein.

**Tabelle 3**

| Wirkeigenschaften von ätherischen Ölen, Pflanzenextrakten sowie aus diesen Pflanzenextrakten isolierter Monosubstanzen | | | |
|---|---|---|---|
| **Eigenschaften** | **Ätherische Öle** | **Pflanzenextrakte** | **Chem.-pharm. wirksame Substanzen** |
| adstringierend | Geranienöl | Gerbstoffe, z.B. | |
| | Zitronenöl | Quercus | |
| | Zypressenöl | Extrakt aus Stipites dulcamara | |
| | | Hamamelisextrakt | |
| Akne | | | Azelain |
| | | | Tretinoin |
| | | | Isotretinoin |
| | | | Adapalen |
| | | | Benzoylperoxid |
| analgetisch | Basilikumöl | Johanniskrautöl | Carbonsäuren z.B.: |
| | Bergbohnenkrautöl | Fructus capsici | • Salicylsäure |
| | Birkenöl | (Capsaicin) | • Diffunisal |
| | Fenchelöl | Beinwellextrakt | • Salicylamid |
| | Fichtennadelöl | Extractum symphyti | • Ethenzamid |
| | Ingweröl | Harpagophytum | • Acetylsalicylsäure |
| | Kamillenöl römisch | Procumbens | • Salsalat |
| | Kampferöl | Weidenrinde | |
| | Lavendelöl extra | Guajakholz | Essigsäurederivate z.B.: |
| | Lavendelsalbeiöl | Arnikaextract | |
| | Lorbeeröl | | • Indometacin / Acemetacin, Proglumetacin |
| | Majoranöl | | |
| | Melissenöl | | |
| | Niaouliöl | | • Diclofenac |
| | Oreganoöl | | • Tolmetin |
| | Patchouliöl | | • Lonazolac |
| | Pfefferöl | | • Fenbufen |
| | Pfefferminzöl | | • Aceclofenac Etofenamat |
| | Rainfarnöl | | |
| | Rosmarinöl | | |
| | Sade-Baumöl | | |
| | Schafgarbeöl | | |
| | Speiklavendelöl | | |
| | Tea Treeöl | | |
| | Thymianöl Wintergrünöl | | |
| Fortsetzung: analgetisch | | | Propionsäurederivate, z.B.: |
| | | | • Ibuprofen |
| | | | • Ketoprofen |
| | | | • Flurbiprofen |
| | | | • Tiaprofensäure |
| | | | • Fenoprofen |
| | | | • Naproxen |
| | | | • Dexketoprofen |
| | | | • Dexibuprofen |
| | | | |
| | | | Heterocyclische Ketoenolsäuren Oxicame: |
| | | | • Piroxicam |
| | | | • Tenoxicam |
| | | | • Metoxicam |
| | | | • Meloxicam |
| | | | • Lornoxicam |
| | | | Anthranilsäure-Derivate: |
| | | | • Mefenaminsäure |
| | | | • Flufenaminsäure |
| | | | • Nifluminsäure |
| | | | |
| Fortsetzung: analgetisch | | | Sonstige Derivate: |
| | | | • Nabumeton |
| | | | • Azapropazon |
| | | | • Aceclofenac |
| | | | • Coffein |
| | | | |
| | | | Pyrazolidione: |
| | | | • Azapropazon |
| | | | • Oxyphenbutazon |
| | | | • Phenylbutazon / Mofebutazon |
| | | | • Azapropazon |
| | | | |
| | | | Weitere Substanzklassen: |
| | | | • Paracetamol |
| | | | • Nifluminsäure |
| | | | • Bufexamac |
| | | | Pyrazolinone: |
| | | | • Propylphenazon |
| | | | • Metamizol |
| | | | |
| | | | Cox-2-Hemmer z.B. |
| | | | • Celecoxib |
| | | | • Rofecoxib |
| | | | • Valdecoxib |
| | | | • Eforicoxib |
| | | | • Parecoxib |
| Neuropathien | | | |
| Neuropatisch | | | • Vitamin-B-Komplex |
| | | | • α-Liponsäure |
| | | | • L'Camithin |
| | | | |
| | | | Periphere Sympatikusblocker: |
| | | | • Clonidin |
| | | | |
| | | | Homöopathische Zubereitung |
| anästhesierend | Kampferöl | | Ester-Lokalanäthetika |
| | Pfefferminzöl | | • Benzocain |
| | Thymianöl | | • Procain (0) |
| | | | • Tetracain (0) |
| | | | • Thymol |
| Fortsetzung anästhesierend | | | Amid-Lokalanästhetika |
| | | | • Prilocain |
| | | | • Mepivacain |
| | | | • Lidocain |
| | | | • Etidocain |
| | | | • Bupivacain |
| | | | • Levobupivacain |
| | | | • Ropivacain |
| | | | • Articain |
| | | | |
| | | | • Fomocain |
| antiallergisch | Schwarzkümmelöl | | Glucocorticoide |
| antibakteriell antiinfektiös | Bayöl | Nachtkerzenöl | Harnstoff |
| | Bergbohnenkrautöl | Neemöl | Thymol |
| | Cajeputöl | Extrakte aus Stipites | Chlorhexidin |
| | Cassiszimtöl | dulcamara | |
| | Cistroseöl | | Antibiotika: |
| | Eukalyptusöl | | • Fusidinsäure |
| | Geranienöl | | • Mupirocin |
| | Gewürznelkenöl | | • Sulfadiazin |
| | Ho-Baumöl | | • Erythromycin |
| | Kamillenöl | | • Clindamycin |
| | Kampferöl | | • Tetracycline |
| | Kiefernöl | | • Medocyclin |
| | Knoblauchöl | | |
| | Lavendelöl | | • Tyrothricin |
| | Lavendelöl extra | | • Gentamycin |
| | Lavendelsalbeiöl | | • Neomycin |
| | Lemongrasöl | | • Bacitracin |
| | Majoranöl | | • Chloramphenicol |
| | Manukaöl | | • Polymyxin |
| | Nelkenöl | | • Kanamycin |
| | Niaouliöl | | |
| | Oreganoöl | | |
| | Patchouliöl | | |
| | Peru-Balsam | | |
| | Petit Grainöl | | |
| | Pfefferminzöl | | |
| | Pfefferöl schwarz | | |
| | Pimentöl | | |
| | Salbeiöl | | |
| | Speiklavendelöl | | |
| | Tea Treeöl | | |
| | Thujaöl | | |
| | Thymianöl | | |
| | Wacholderöl | | |
| | Ysopöl | | |
| | Zimtöl | | |
| | Zitronenöl | | |
| antihämorrhagisch | Cistroseöl | Hamamelisextrakt | |
| antihistaminisch | Schwarzkümmelöl | | Glucocorticoide |
| Antihyperhidrotisch | | Salbei | Kampfersäure |
| | | Walnussblätter | Methenamin |
| | | Eichenrinde | Aluminium chlorat. |
| | | Gerbstoffe z.B. | hexahydrat |
| | | Eichenrinde | |
| antikoagulierend | Immortellenöl | | Hirudin |
| | Strohblumenöl | | Hirudinderivate |
| | Zimtöl | | Heparine, insbesondere auch niedermolekulare |
| | Lavendelöl | | |
| | Lorbeeröl | | |
| | Zitronenöl | | |
| antimykotisch | Bayöl | Neemöl | Azolderivata: |
| | Pimentöl | | |
| | Bergbohnenkrautöl | Extrakte aus Stipites dulcamara | • Clotrimazol |
| | Cassiszimtöl | | • Bifonazol |
| | Eukalyptusöl | | • Econaxol |
| | Geranienöl | | • Fenticonazol |
| | Gewürznelkenöl | | • Isoconazol |
| | Ho-Baumöl | | • Oxiconazol |
| | Kampferöl | | • Sertaconazol |
| | Zimtöl | | • Tioconazol |
| | Lavendelöl | | • Miconazol |
| | Lavendelöl extra | | • Ketoconazol |
| | Manukaöl | | • Itraconazol |
| | Nelkenöl | | • Fluconazol |
| | Niaouliöl | | • Voriconazol |
| | Oreganoöl | | • Sertaconazol |
| | Patchouliöl | | |
| | Pfefferminzöl | | |
| Fortsetzung antimykotisch | Pimentöl | | Squalenepoxidosehemmer z.B.: |
| | Rosmarinöl | | |
| | Salbeiöl | | • Terbinafin |
| | Speiklavendelöl | | • Naftifin |
| | Tagetesöl | | |
| | Tea Treebaumöl | | Morpholine z.B.: |
| | Thymianöl | | • Amorolfin |
| | | | |
| | | | Sonstige antimykotisch wirksame Substanzen z.B.: |
| | | | • Amphotericin B |
| | | | • Giseofulvin |
| | | | • Flucytosin |
| | | | • Ciclopirox |
| | | | • Nystatin |
| | | | • Natamycin |
| | | | • Thiocarbonate |
| antiödematös aquaretisch entstauend entwässernd ödemprotektiv | Baldrianöl | Aesculus hippocastanum | |
| | Basilikumöl | | |
| | Fenchelöl | Ruscus acculeatus | |
| | Geranienöl | Melilotus officinalis | |
| | Kampferöl | Fagopyrum esculentum | |
| | Kiefernöl | | |
| | Lorbeeröl | Roter | |
| | Majoranöl | Weinlaubextrakt | |
| | Patchouliöl | Solidago virgaurea | |
| | Pfefferöl | Brennessel | |
| | Rosmarinöl | | |
| | Sandelholzöl | | |
| | Schwarzkümmelöl | | |
| | Texas-Zedernöl | | |
| | Thujaöl | | |
| | Wacholderöl | | |
| | Zypressenöl | | |
| antioxidativ | | Flavonoide | Selen |
| | | Anthocyane | Mangan |
| | | Proanthoxycyanidine | Kupfer |
| | | Carotinoide | |
| | | | L' Glutathion |
| | | Betacarotin: | L' Cystein |
| | | Lycopin | |
| | | Zeaxanthin | Coenzym Q 10 |
| | | Vitamin A, C und E | a-Liponsäure |
| antiparasitisch | Pfefferminzöl | Neemöl | Crotamiton |
| | Zimtöl | | Permethrin |
| | Tea Treeöl | | Benzylbenzoat |
| | | | Allethrin |
| antiphlogistisch | Basilikumöl | Melilotus officinalis | Stereoidale |
| | Benzoeharz | Ruscus acculeatus | Antiphlogistika wie |
| | Birkenöl | Aesculus | Glukocorticoide |
| | Campher | hippocastanum | |
| | Eukalyptusöl | Johanniskrautöl | Bufexamac |
| | Fichtennadelöl | Ringelblume | Glycyrrhetinsäure |
| | Galbanumöl | Aloe vera | |
| | Johanniskrautöl | Jojoba | Thymol |
| | Kamillenöl blau | Nachtkerzenöl | Cavacrol |
| | Kamillenöl römisch | Borretschsamenöl | Campher |
| | Latschenkiefernöl | Cardiospermum | Eugenol |
| | Lavendelöl | helicacabum | Zimtaldehyd |
| | Lavendelöl extra | Gerbstoffe z.B. aus Quercus und | Kapsaicin |
| | Lemongrasöl | | |
| | | Synthetica | |
| **Fortsetzug** antiphlogistisch | Manukaöl | Extrakte aus Stipites | |
| | Myrrheöl | dulcamara | |
| | Nelkenöl | Extrakte aus | |
| | Patchouliöl | Symphyti | |
| | Petit Grainöl | Hamamelisextrakt | |
| | Peru-Balsam | Kamille | |
| | Rosmarinöl | | |
| | Schafgarbeöl | Arnikaöl | |
| | Schwarzkümmelöl | Propolis | |
| | Sumpfporstöl | | |
| | Tea Treeöl | | |
| | Thymianöl | | |
| | Weihrauchöl | | |
| | Wintergrünöl | | |
| | Ysopöl | | |
| | Zimtöl | | |
| antirheumatisch | Birkenöl | Fructus capsici | Siehe Auflistung analgetisch chem.-pharm. Wirksame Substanzen |
| | Kampferöl | Capsaicin | |
| | Manukaöl | Nicotinsäure | |
| | Rosmarinöl | Salicylsäureester | |
| | Wacholderöl | Cortex Salicis | |
| | Wintergrünöl | Urtica dioica | Salicin |
| | Zimtöl | Urtica urens | |
| antiseptisch | Benzoeharz | | |
| | Bergamotteöl | | |
| | Bergbohnenkrautöl | | |
| | Galbanumöl | | |
| | Geranienöl | | |
| | Kampferöl | | |
| | Weißtannenöl | | |
| antispasmodisch | Basilikumöl | | |
| | Birkenöl | | |
| | Lavendelsalbeiöl | | |
| | Lorbeeröl | | |
| | Majoranöl | | |
| | Peru-Balsam | | |
| | Petit Grainöl | | |
| | Wintergrünöl | | |
| antiviral | Basilikumöl | Extractum | • Aciclovir / Valciclovir |
| | Bayöl | Podophyllum | |
| | Pimentöl | (Podophyllin) | • Penciclovir / Famciclovir |
| | Cajeputöl | Extractum Melissae | |
| | Cassiazimtöl | Fructus Capsici | • Idoxuridin / Bivudin |
| | Cistroseöl | Capsaicin | |
| | Eukalyptusöl | | • Trifluridin |
| | Gewürznelkenöl | | • Vidarabin |
| | Ho-Baumöl | | • Tromantadin |
| | Kampferöl | | • Foscarnet |
| | Zimtöl | | • Interferon-beta |
| | Lemongrasöl | | • Podophyllotoxin |
| | Melissenöl | | |
| | Myrrheöl | | |
| | Niaouliöl | | |
| | Oreganoöl | | |
| | Pfefferöl | | |
| | Pfefferminzöl | | |
| | Pimentöl | | |
| | Salbeiöl | | |
| | Speiklavendelöl | | |
| | Tea Treeöl | | |
| | Thujaöl | | |
| | Thymianöl | | |
| | Ysopöl | | |
| | Zitronenöl | | |
| bindegewebsregenerierend | | Allium cepa | • Heparin |
| | | Centella asiatica | • Asiaticosid |
| Errektile Dysfunktion | | | Alfrostadil (PGE 1) |
| | | | Sildenafilcitrat |
| | | | Vardenafil |
| | | | Tadalafil |
| | | | Durchblutungsfördernd wie |
| | | | Benzylnicotinat |
| epithelisierend | Bergamotteöl | | |
| | Geranienöl | | |
| | Immortellenöl | | |
| | Strohblumenöl | | |
| | Lavendelöl | | |
| | Lavendelöl extra | | |
| | Myrrheöl | | |
| | Patchouliöl | | |
| | Pfefferminzöl | | |
| | Sandelholzöl | | |
| | Schafgarbeöl | | |
| | Tea Treeöl | | |
| | Thujaöl | | |
| | Weihrauchöl | | |
| feuchtigkeitsbindend | | Avocadoöl | Harnstoff |
| | | Wildrose | Glycerin |
| | | Aloe vera | Glycine |
| gefäßerweiternd | Lemongrasöl | | Nitropräparate |
| Haarausfall | | | Finasterid |
| | | | Minoxidil |
| hautnährend | Angelikaöl | Wildrose | Aminosäuren |
| hautpflegend | Baldrianöl | Mandelöl | Vitamine |
| hautregenerierend | Benzoeharz | Weizenkeimöl | |
| | Bergamotteöl | Avocadoöl | |
| | Geranienöl | Aloe vera | |
| | Manukaöl | Borretschsamenöl | |
| | Myrrheöl | Jojobaöl | |
| | Vetiveröl | Mandelöl | |
| | | Sheabutter | |
| | | Wildrose | |
| herzstärkend | | Arnikablüten | |
| | | Weißdornextract | |
| hyperämisierend | Cassiazimtöl | Arnikaöl | Nicotinsalicylsäureester |
| | Birkenöl | Erdnußöl | |
| Durchblutungsfördernd | Ingweröl | Olivenöl | Capsaicin |
| | Kampferöl | | Capsaicinoide |
| | Kiefernöl | | Coffein |
| | Oreganoöl | | Benzylnicotinat |
| | Pfefferöl schwarz | | Nonivamid |
| | Rosmarinöl | | Nicobexil |
| | Sade-Baumöl | | Methylsalicylat |
| | Vetiveröl | | |
| | Eucalyptusöl | | |
| | Terpentinöl | | |
| | Campher | | |
| | Weißtannenöl | | |
| | Zimtöl | | |
| Immunmodulatorisch | Kampferöl | Extrakte aus Stipites | |
| | Zimtöl | dulcamara | |
| | Lemongrasöl | Viola tricolor | |
| | Melissenöl | Similax-Arten | |
| | Niaouliöl | Phytolacca americana | |
| | Oreganoöl | Glyzyrrhiza glabra | |
| | Patchouliöl | Mistelextrakt | |
| | Tea Treeöl | Bryonia alba | |
| | Thymianöl | Echinaceaextrakt | |
| | Weihrauchöl | | |
| juckreizstillend | Lavendelöl | Melilotus officinalis | Bufexamac |
| | Manukaöl | Ruscus amleatus | Synthetische |
| | | Fructus capsici | Gerbstoffe |
| | | Capsicum (Capaicin) | Glycyrrhetinsäure |
| | | Borretschsamenöl | |
| | | Avocadoöl | |
| | | Nachtkerzenöl | |
| | | Wildrosenöl | |
| | | Gerbstoffe z.B. aus Quercus | |
| | | Hamamelisextrakt | |
| Keratolytisch | | Mahonia | Vitamin-A-Säure |
| Antipsoriatisch | | aquitolium | Harnstoff |
| | | | Salicylsäure |
| | | | Tazaroten |
| kühlend | Pfefferminzöl | Menthol | Menthol |
| mitosehemmend | | | Colchicin |
| | | | Colchicinderivate |
| muskelrelaxierend | | | Periphere z.B.: |
| | | | |
| | | | Stabilisierend: |
| | | | • Tubocurarinchlorid |
| | | | • Alcuroniumchlorid |
| Fortsetzung muskelrelaxierend | | | Depolarisationsverhindernd: |
| | | | • Pancuroniumbromid |
| | | | • Vecuroniumbromid |
| | | | • Atracuriumbesilat |
| | | | • Mivacuriumchlorid |
| | | | • Rocuroniumbromid |
| | | | • Cisatracuriumbesilat |
| | | | Repolarisierend z.B.: |
| | | | • Suxamethoniumchlorid |
| | | | Senkung des erhöhten Skelettmusekltonus: |
| | | | • Dentrolen |
| | | | Irreversible |
| | | | Hemmung der neuromuskulären |
| | | | Übertragung: |
| | | | • Clostridium |
| | | | • Botulinum |
| | | | • Botulin und Abkömmlinge |
| | | | Cotylinum (Botox) |
| | | | Na-Kanalhemmer wie Tolperison |
| | | | Lokalannesthetika |
| | | | Chininsulfat |
| phlebotonisch | Basilikumöl | Hamamelisextrakte | Sparteinsulfat |
| | Cajeputöl | Ruscus acculeatus | Digitoxin |
| | Geranienöl | Melilotus albus | Heparin |
| | Melissenöl | Rotes Weinlaub | Mutterkomalkaloide |
| | Niaouliöl | Aesculus | insbesondere |
| | Rainfarnöl | hypocastanum | Dihydroergotamin |
| | Tea Treeöl | Melilotus officinalis | Diosmin |
| | Zitronenöl | Centellaextrakt | Flavonoidderivate |
| | Zypressenöl | Fagopyrum | |
| | | escalentum | |
| | | Pinus maritima | |
| Schuppung | Borretschsamenöl | | |
| hemmend | Nachtkerzenöl | | |
| sedierend | | Extractum valerianae | |
| | | Melissenöl | |
| spasmolytisch | Pfefferminzöl | | |
| | Campher | | |
| | Fichtennadelöl | | |
| vasodilatatorisch | Birkenöl | Weißdornextrakt | Nitroclycerin |
| | Wintergrünöl | | Benzylnicotinat |
| wundheilend | Bergamotteöl | Wildrose | |
| antitraumatisch | Galbanumöl | Sheabutter | |
| | Geranienöl | Olivenöl | |
| | Johanniskrautöl | Nachtkerzenöl | |
| | Kamillenöl | Arnikaöl | |
| | Thujaöl | Avocadoöl | |
| | | Aloe vera | |
| | | Jojobaöl | |
| | | Oleum Calendulae | |
| | | Oleum Chamomilla | |
| | | Extractum Hamamelis | |
| | | Oleum Hyperici | |
| | | Gerbstoffe | |
| | | Extractum Calendulae | |
| | | Extractum Symphyti | |
| | | Extractum Hyperici | |

Durch Lösen oder Dispergieren der oben genannter Basisstoffe, ätherischen Öle, Pflanzenextrakte und /oder synthetischen Monosubstanzen in einer Mikroemulsion können unter anderem folgende Arzneimittel formuliert werden:
Arzneimittel zur Behandlung äußerer, rheumatischer Beschwerden, die analgetisch, antiphlogistisch, hyperämisierend, und / oder spasmolytisch wirkende Arzneistoffe aufweisen.
Arzneimittel zur Behandlung des komplexen, peripheren Schmerz-Syndroms, die analgetisch, antioxidativ, antiphlogistisch, spasmolytisch, muskekelaxierend, hyperämisierend, und / oder lokalanästhetisch wirkende Arzneistoffe aufweisen.
Arzneimittel zur Behandlung von Wunden, Prellungen, Zerrungen, Sportverletzungen und Oedemen, die wundheilend, analgetisch, thrombolytisch, fibrinolytisch, epithelisierend, antikoagulierend, antiphlogetisch, antibakteriell, antiviral, antimykotisch, aquaretisch, hauternährend und / oder antitraumatisch wirkende Arzneistoffe aufweisen.
Arzneimittel zur Behandlung von chronischen Wunden, die antioxidativ, analgetisch, antiphlogistisch, und / oder heilend wirkende Arzneistoffe aufweisen.
Arzneimittel zur Behandlung von Haarausfall.
   Arzneimittel zur Behandlung bei errektiler Dysfunktion.
   Arzneimittel zur Behandlung bei übermäßiger Schweißsektion.
Arzneimittel zur Behandlung von Neuralgien, die analgetisch und / oder lokalanästhetisch wirkende Arzneistoffe aufweisen.
Arzneimittel zur Behandlung von diabetischen Neuropathien, die analgetisch, hyperämisierend, juckreizstillend und / oder brennenlindernd wirkende Arzneistoffe aufweisen.
Arzneimittel zur Behandlung von Varikosis, Phlebitis, die phlebotonisierend, ödemprotektiv, juckreizstillend, antikoagulierend, fibrinolytisch, antispastisch, aquaretisch, entstauend, antioxidativ und / oder hämolytisch wirkende Arzneistoffe aufweisen.
Arzneimittel zur Behandlung von Hämorrhoiden, die phlebotonisch, aquaretisch, und / oder epithelisierend wirkende Arzneistoffe aufweisen.
Arzneimittel zur Behandlung von akuten Gichtanfällen, die mitosehemmend, antiphlogistisch, antioxidativ und / oder aquaretisch wirkende Arzneistoffe aufweisen.
Arzneimittel zur Behandlung von Mykosen, die antimykotisch wirkende Arzneistoffe aufweisen.
Arzneimittel zur Behandlung von Neurodermitis und oder Ekzemen, die antiphlogistisch, juckreizstillend, immunmodulierend, hautregernierend, antioxidativ, adstringierend und / oder antiallergisch wirkende Arzneistoffe aufweisen.
Arzneimittel zur Behandlung von Keratosen, die keratolytisch wirkende Arzneistoffe aufweisen.
Arzneimittel zur Behandlung von Psoriasis, die keratolytisch, antiphlogistisch, juckreizstillend, hautregenerierend und / oder antioxidativ wirkende Arzneistoffe aufweisen.
Arzneimittel zur Behandlung von Akne, die keratolytisch, antibakteriell, antiphlogistisch, antioxidativ und / oder wundheilend wirkende Arzneistoffe aufweisen.
Arzneimittel zur Behandlung von viralen Infektionen, die antiviral, analgetisch, antiphlogistisch, keratolytisch und / oder antioxidativ wirkende Arzneistoffe aufweisen.
Arzneimittel zur Behandlung von Hämatomen, die fibrinolytisch wirkende Arzneistoffe aufweisen.
Arzneimittel zur Behandlung von Couperose, die antiphlogistisch und / oder antioxidativ wirkende Arzneistoffe aufweisen.
Arzneimittel zur Behandlung von Krätze, die antiparasitär und / oder juckreizstillend wirkende Arzneistoffe aufweisen.
Arzneimittel zur Behandlung von degenerierter Haut, die antiphlogistisch, antimikrobiell, ernährend und / oder lokalanaesthetisch wirkende Arzneistoffe aufweisen.
Arzneimittel zur Behandlung von Angina Pectoris - Brustschmerzen, die hyperämisierend, und / oder spasmolytisch wirkende Arzneistoffe sowie schmerzreizunterbrechende Arzneistoffe aufweisen.
Arzneimittel zur Behandlung von Pruritus, die kühlend, lokal anaesthesierend, analgetisch, antiphlogistisch und / oder adstringierend wirkende Arzneistoffe aufweisen.
Arzneimittel zur Behandlung von Narben und Keloiden, die bindegewebsregulierende Arzneistoffe aufweisen.

In einer besonders bevorzugten Ausführungsform können mehrere Arzneimittel auf Basis der gleichen Mikroemulsionen zu Kombinationspräparaten kombiniert werden.

Die Konzentration der Arzneistoffe in den Mikroemulsionen ergibt sich aus den empfohlenen Leitlinien und Richtlinien der Therapie und der in der Praxis handhabbaren Menge an Mikroemulsion.

Konkret kann die Konzentration des Arzneistoffs in der Mikroemulsion zwischen 0 und 100 % betragen, wobei Konzentrationen zwischen 10⁻⁸ % und 50 % bevorzugt und zwischen 10⁻⁶ und 5 % besonders bevorzugt sind.

Durch Anreicherung dieser und weiterer Arzneimittel auf Basis von Mikroemulsionen mit Sauerstoff werden erfindungsgemäße Arzneimittel zur perkutanen Verabreichung erhalten. Diese Anreicherung kann bei der Herstellung der Arzneistoffe erfolgen.

Unter mit Sauerstoff angereicherten Mikroemulsionen werden Mikroemulsionen verstanden, die in einem geeigneten Verfahrensschritt mit Sauerstoff angereichert werden. Ein solcher Verfahrensschritt stellt beispielsweise das Zerstäuben der Mikroemulsion in sauerstoffhaltiger Atmosphäre dar. Dabei ist der Sauerstoffgehalt dieser Atmosphäre vorzugsweise größer als 25 Volumenprozent, besonders bevorzugt größer 50 Volumenprozent und insbesondere größer 90 Volumenprozent.

Vorzugsweise weist die mit Sauerstoff angereicherte Mikroemulsion eine Sauerstoffkonzentration von größer 10⁻³ mol/L, insbesondere von größer 5x 10⁻³ mol/L, auf.

Um zu verhindern, dass bei der Herstellung mit Sauerstoff angereicherte Mikroemulsionen den Sauerstoff bis zum Applikationszeitpunkt wieder abgeben, sind diese Mikroemulsionen vorzugsweise gasdicht verpackt.

Auch andere Zusätze zu diesen Arzneimitteln, sowie zu anderen Arzneimitteln auf Basis von Mikroemulsionen, welche die Sauerstoffversorgung der Haut verbessern, führen zu erfindungsgemäßen Arzneimitteln.

Beispiele für Zusätze, welche die Sauerstoffversorgung der Haut verbessern sind natürliche Sauerstoffträger, wie Myoglobin und / oder Hämoglobin sowie Fluorcarbone.

Eine Anreicherung der Mikroemulsion mit Sauerstoff kann auch direkt beim Verabreichen der Mikroemulsion mit Hilfe eines Applikationssystems zur perkutanen Verabreichung von Arzneistoffen, aufweisend mindestens einen Arzneistoff enthaltende Mikroemulsion und eine Vorrichtung zum Zerstäuben der Mikroemulsion, erfolgen. Dabei ist die Anreicherung mit Sauerstoff direkt beim Verabreichen bevorzugt.

In einem solchen erfindungsgemäßen Applikationssystem ist vorzugsweise die Mikroemulsion in einem Behälter enthalten, der an eine Zerstäubungseinheit angeschlossen ist, wobei an die Zerstäubungseinheit eine unter Druck stehende Gasquelle, vorzugsweise ausgewählt aus Sauerstoff, Luft, Stickstoff, Edelgas, angeschlossen ist, und die Mikroemulsion durch die Wirkung des unter Druck stehenden Gases zerstäubt wird.

Ebenso ist es möglich die Barrierefunktion des Stratum corneum durch Auftragen einer erfindungsgemäßen Mikroemulsion ohne Arzneistoffe, die mit Sauerstoff angereichert ist und / oder die einen Zusatz aufweist, der die Sauerstoffversorgung der Haut verbessert, zeitweise aufzuheben. Dies gelingt auch durch Auftragen einer geeigneten Mikroemulsion ohne Arzneistoffe, beispielsweise mit einem erfindungsgemäßen Applikationssystem. Die zu verabreichenden Arzneistoffe werden dann in einem weiteren Schritt auf die betreffende Hautpartie aufzutragen.

Bei Anwendung des erfindungsgemäßen Systems, wobei ein sauerstoffhaltiges Treibgas verwendet wird, reichern sich die Mikroemulsionen, die appliziert werden, direkt vor deren Eintritt in das Stratum Corneum mit Sauerstoff an. Dies führt zu einer Erhöhung des Sauerstoffpartialdrucks auf der Gewebsseite des Stratum corneums und damit zu einer Stimulation der kutanen Mikrozirkulation und zu einem verbesserten transdermalen Transport der Arzneistoffe. Ebenso kann der transdermale Transport von Arzneistoffen beispielsweise auch durch einen erhöhten Transmembrandruck, hier verursacht durch die Erhöhung der Sauerstoffkonzentration auf der Gewebeseite des Stratum Corneum, mitverursacht sein.

Die Anwendung dieses Systems ist besonders geeignet bei Arzneimitteln, wie in den Ansprüchen 4-8 genannt bzw. bei solchen, die oxidativ empfindliche Substanzen aufweisen und daher erst direkt vor der Applikation mit Sauerstoff angereichert werden können.

Es ist möglich mit einem erfindungsgemäßen Applikationssystem die Arzneistoffdosis, die appliziert werden soll, genau zu dosieren, wodurch dann auch die maximale Tagesdosis appliziert werden kann. Dazu wird eine Mikroemulsion, welche die maximale Tagesdosis eines oder mehrerer Arzneistoffe aufweist, in das System zur perkutanen Verabreichung von Arzneistoffen gegeben und mit diesem System einem Patienten verabreicht.

Ein weiterer Effekt der Zerstäubung, der zu einem verbesserten transdermalen Transport von Arzneimitteln beitragen kann, ist der Spreiteffekt. Dieser beruht auf der feinen Verteilung der Tröpfchen beim Zerstäuben. Dadurch gelangt die Mikroemulsion in Form kleiner Tröpfchen besser in Vertiefungen, Falten und Öffnungen in der Haut.

Die oben genannten Arzneimittel auf Basis von Mikroemulsionen bilden bevorzugte Ausführungsformen eines Systems zur perkutanen Verabreichung von Arzneistoffen im Rahmen dieser Erfindung.

Nachfolgend wird das erfindungsgemäße Applikationssystem zur Zerstäubung von flüssigen Arzneimitteln zur perkutanen Arzneimittelverabreichung näher erläutert.

Die Umsetzung des Applikationssystems erfolgt erfindungsgemäß mit den in den Patentansprüchen angegebenen Merkmalen.

Bei dem erfindungsgemäßen Applikationssystem zur Zerstäubung von flüssigen Arzneimitteln zur perkutanen Arzneimittelverabreichung, wird ein genau dosiertes flüssiges Arzneimittel, insbesondere eine den Arzneistoffenthaltende Mikroemulsion, zur Auftragung auf die Haut mittels eines Treibgases, vorzugsweise hochkonzentrierter Sauerstoff, unter Druck durch eine Mikrodosierdüse gepresst und vorzugsweise durch Ausnutzung einer durch den Venturi-Effekt begründeten Sogwirkung feinst zerstäubt.

Mit der Mikrodosierdüse des Applikationssystems ist ein Spektrum von Tröpfchengrößen generierbar, wobei der Austrittsquerschnitt der Mikrodosierdüse durch eine positionierbare Nadelspitze variiert und somit die Tröpfchengröße verändert werden kann. Der durch die Zerstäubung erzielbare Durchmesser der Tröpfchen liegt im Nanometerbereich, wobei die gemessene mittlere Tröpfchengröße weniger als 1 µm, vorzugsweise weniger als 400nm, insbesondere weniger als 300nm beträgt. Die Reproduzierbarkeit des Tröpfchengrößenspektrums mit dem Applikationssystem ist durch berührungsfreie und laseroptische Messmethoden nachweisbar.

Aus der Vielzahl der verschiedenen Tropfen einer Zerstäubungsflüssigkeit können mittels der Laser-Beugungsspektroskopie die einzelnen Tröpfchengrößen und deren Häufigkeit bestimmt werden. Dabei wird das monochromatische Licht eines Laserstrahls durch die einzelnen Tropfen einer Zerstäubungsflüssigkeit mehr oder minder stark gebeugt, wobei die auf einem Detektor befindlichen Fotomultiplier unterschiedliche Impulse und Intensitäten registrieren. Eine nachgeschaltete Elektronik mit spezieller Software wertet diese aus und errechnet hieraus die tatsächliche Tröpfchengrößenverteilung.

Mit dem erfindungsgemäßen Applikationssystem können alle zubereiteten und zu zerstäubenden flüssigen Arzneimittel, vorzugsweise Arzneimittel auf Basis von Mikroemulsionen, mit bestimmten rheologischen Eigenschaften, wie z.B. Viskosität, Flüssigkeitsdichte, Oberflächenspannung, aber insbesondere unterhalb einer gewissen dynamischen Viskosität auf den Ort der zu behandelnden Haut aufgesprüht werden.

Neben hochkonzentriertem Sauerstoff sind als Treibgas alternativ Luft, Stickstoff oder ein Edelgas (Helium, Argon) anwendbar. Dabei wird unter hochkonzentriertem Sauerstoff ein Gas verstanden, das mit mindestens 90 Vol % Sauerstoff angereichert ist Werden Treibgase verwendet, die keinen Sauerstoff enthalten, ist die Mikroemulsion bereits mit Sauerstoff angereichert und / oder enthält Zusätze, welche die Sauerstoffversorgung der Haut verbessern.

Bei der Zerstäubung wird das zubereitete Arzneimittel vom Treibgas umgeben und mit diesem vermischt. Hierbei löst sich das Treibgas in dem flüssigen Arzneimittel unter Druck, wodurch eine positive Eigenschaft der die Haut in Verbindung mit Sauerstoff stimulierenden flüssigen Wirksubstanz erzeugt werden kann.

Ein positiver Effekt der äußerst feinen Zerstäubung ist die bei der perkutanen Arzneimittelverabreichung, aufgrund der Verdunstungskälte, angenehm kühlende Wirkung des fein zerstäubten Arzneimittels.

Aufgrund der Reaktivität des hochkonzentrierten Sauerstoffs sind für die einzelnen Komponenten des Applikationssystems Materialien zu verwenden, die dem Sauerstoffstandhalten, wie z.B. Glas, spezielle klinisch zugelassene Kunststoffe oder Edelstahl.

Bei der Zerstäubung der Mikroemulsion wird vorteilhaft, je nach Tagesdosis und zu behandelnder Körperpartie, ein Volumenstrom von 1,5 bis 5 ml/ 20 min bzw. 4,5 bis 15 ml/h durch den Austrittsquerschnitt der Mikrodosierdüse durchgesetzt.

Das Treibgas kann einem Gasbehälter entnommen und dem Applikationssystem über eine Schlauchverbindung zugeführt werden. Der Gasbehälter selbst kann Bestandteil einer Sauerstoffaufbereitungsanlage (O₂-Anlage) sein, bei der aus Umgebungsluft Sauerstoff gewonnen und in diesem angereichert wird.

Alternativ ist in einer weiteren Ausführungsform von Applikationssystem und Gasquelle, auch ein autarker Gasbehälter oder ein Gasanschluss in einer Klinik denkbar.

In einer bevorzugten Ausführungsform der Erfindung ist das Applikationssystem als autarkes, mit flüssigem Arzneimittel gefülltes und an ein Treibgassystem angeschlossenes System ausgebildet.

Nachfolgend wird unter Bezugnahme der Figuren 1, 2 und 3 das erfindungsgemäße Applikationssystem zur perkutanen Arzneistoffverabreichung, insbesondere von flüssigen Arzneimitteln auf Basis von Mikroemulsionen, näher erläutert. Es zeigen:
- Fig. 1: eine schematische Darstellung eines Applikationssystems,
- Fig. 2: eine vergrößerte schematische Darstellung des düsennahen Bereiches Applikationssystems nach Fig. 1 und dessen Funktionsprinzip, und
- Fig. 3: eine schematische Darstellung eines weiteren Applikationssystems,
- Fig. 4: eine schematische Darstellung eines weiteren Applikationssystems.

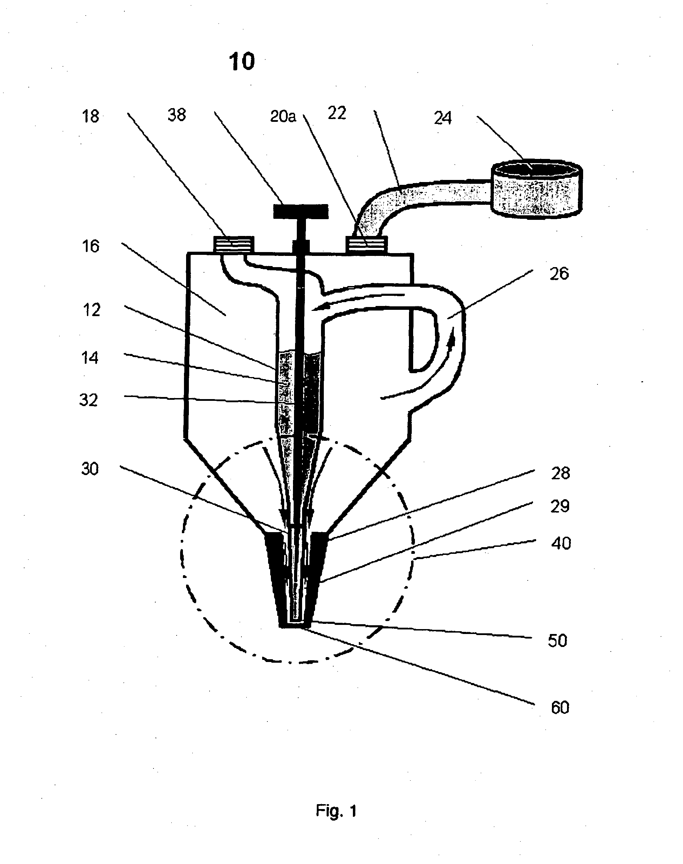

Fig. 1 zeigt ein Applikationssystem 10 in vereinfachter schematischer Darstellung der einzelnen Komponenten. Das Applikationssystem umfasst ein Arzneimittelreservoir 12, das in einem Gasreservoir 16 des Applikationssystems 10 angeordnet ist. Das Arzneimittelreservoir 12 ist an seinem Ende, im düsennahen Bereich 40 des Applikationssystems 10, zu einer Kapillare verjüngt.

Je nach zu verabreichender Tagesdosis, befinden sich im Arzneimittelreservoir 12 zwischen 1,5 bis 5 ml eines Arzneimittels 14. Das obere Ende des Arzneimittelreservoirs 12 und das Gasreservoir 16 des Applikationssystems 10 sind in der Gebrauchslage gesehen koaxial ausgebildet und über eine By-passleitung 26 bzw. ein Ausgleichsrohr 26 miteinander verbunden. Ebenfalls am oberen Ende befinden sich ein Einlass 18 zur Befüllung eines Arzneimittels 14 in das Arzneimittelreservoir 12 und ein Einlass 20 zur Befüllung des Gasreservoirs 16 mit einem Treibgas. Das Gasreservoir 16 des Applikationssystems 10 ist über eine Schlauchverbindung 22 an einen Gasbehälter 24 angeschlossen. Im düsennahen Bereich 40 ist das Applikationssystem 10 als Rotationskörper, mit sich in Richtung des Düsenaustritts 50 verjüngendem Querschnitt, ausgebildet.

Das Arzneistoffreservoir 12 schließt mit seinem verjüngten Ende an die Zerstäubungsdüse 30 an, die innerhalb des Düsenkopfes 28 angeordnet ist. Der Düsenkopf 28 weist entlang seiner Rotationsachse Öffnungen 29 auf, über die das Gasreservoir 16 mit der Umgebung in Strömungsverbindung steht. Eine in die Zerstäubungsdüse 30 hineinragende Nadel 32, die im oberen Teil des Gasreservoirs 16 geführt ist, verengt deren kreisförmigen Querschnitt. Durch Drehung eines Rädelkopfes 34 ist die Nadel vertikal positionierbar und dadurch die Querschnittsverengung der Zerstäubungsdüse 30 einstellbar.

Im folgenden wird die Funktionsweise des in Fig. 1 dargestellten Applikationssystems 10 zur Zerstäubung eines zubereiteten Arzneimittels zur perkutanen Arzneimittelverabreichung näher beschrieben.

Je nach Größe der Fläche der zu behandelnden Körperpartie, wird ein genau dosiertes flüssiges Arzneimittel 14, insbesondere ein flüssiges Arzneimittel auf Basis einer Mikroemulsion, vorzugsweise 1,5 bis 5 ml, in das Arzneimittelreservoir 12 über den Arzneimittelreservoireinlass 18 des Applikationssystems 10 eingefüllt.

Zur Zerstäubung des flüssigen Arzneimittels 14 wird das Gasreservoir 16 mit Treibgas , vorzugsweise Sauerstoff, stetig gefüllt, wodurch sich im geschlossenen Gasreservoir 16 ein Überdruck aufbaut. Das Treibgas wird dem Gasbehälter 24 entnommen und unter einem vorbestimmten Druck, im Beispiel ca. 2 bar, dem Applikationssystem 10 zugeführt. Hierzu wird das Gasreservoir 16 über eine Schlauchverbindung 22 an einen Gasanschluss 20 des Applikationssystems 10 angeschlossen.

Durch den Überdruck im Gasreservoir 16 wird das Treibgas bis zur Austrittsstelle 50 der Zerstäubungsdüse 30 (Mikrodosierdüse) befördert. Da das Gasreservoir 16 des Applikationssystems 10 im düsennahen Bereich 40 als Rotationskörper, mit sich in Richtung des Düsenaustritts 40 verjüngendem Querschnitt, ausgebildet ist, wird das Treibgas durch den Überdruck im Gasreservoir 16 in Flussrichtung beschleunigt. Der sich infolge der Querschnittsverengung einstellende Staudruck innerhalb des Gasreservoirs 16, wird über eine By-passleitung 26 zur Erzeugung des Vortriebs des flüssigen Arzneimittels 14 auf das Arzneimittelreservoir 12 umgeleitet, wobei der Staudruck das flüssige Arzneimittel durch die Zerstäubungsdüse 30 presst. Hierdurch wird ein gleichmäßiger Vortrieb gewährt.

Das sich im düsennahen Bereich 40 verjüngende Ende des Arzneimittelreservoirs im Inneren des Gasreservoirs 16 ist derart ausgebildet, dass ein Abriss des flüssigen Arzneimittels unterbunden wird.

Die Öffnungen 29 im Inneren des Düsenkopfes 28 gewährleisten, dass das in Richtung des sich verjüngenden Rotationskörpers 16 beschleunigte Treibgas die Zerstäubungsdüse 30 bis zur Austrittsstelle 50 des Düsenkopfes 28 umströmt.

An der Austrittsstelle 40 der Zerstäubungsdüse 30 angelangt, wird das flüssige Arzneimittel durch den sich in der Austrittsstelle einstellenden Unterdruck (Venturi-Effekt) angesaugt und dabei zerstäubt.

Bei der Zerstäubung wird das zubereitete Arzneimittel 14 vom Treibgas umgeben und mit diesem vermischt. Hierbei löst sich das Treibgas in dem flüssigen Arzneimittel 14. Hierdurch ergibt sich eine verstärkte Wirkung des flüssigen Arzneimittels 14 auf die Mikrozirkulation, insbesondere bei einem flüssigen Arzneimittel auf Basis von Mikroemulsionen, was die perkutane Arzneistoffverabreichung ergeben kann. Der Tröpfchengrößendurchmesser bei der Zerstäubung des flüssigen Arzneimittels 14 kann über die Nadel 32 im Inneren der Zerstäubungsdüse 30 variiert werden, indem die Nadel 32 durch Drehung des Rändelkopfes 38 fein positioniert wird. Wird die Zerstäubungsdüse 30 vollkommen durch die Nadel 32 verschlossen, so dass der Massendurchsatz des flüssigen Arzneimittels 14 durch die Zerstäubungsdüse 30 unterbunden ist, so kommt die Zerstäubung des Arzneimittels zum Erliegen. Es fließt sodann lediglich Treibgas durch die Austrittsstelle 60 des Düsenkopfes 28, aufgrund der im Inneren des Düsenkopfes 28 angeordneten Öffnungen 29 entlang der Zerstäubungsdüse 30.

Andererseits kann in einer weiteren nicht gezeigten Ausführungsform eine Düse mit einem vorbestimmten Innendurchmesser ohne Einstellnadel eingesetzt werden, wenn hierdurch das bereits gewünschte Tröpfchenprofil erreicht wird.

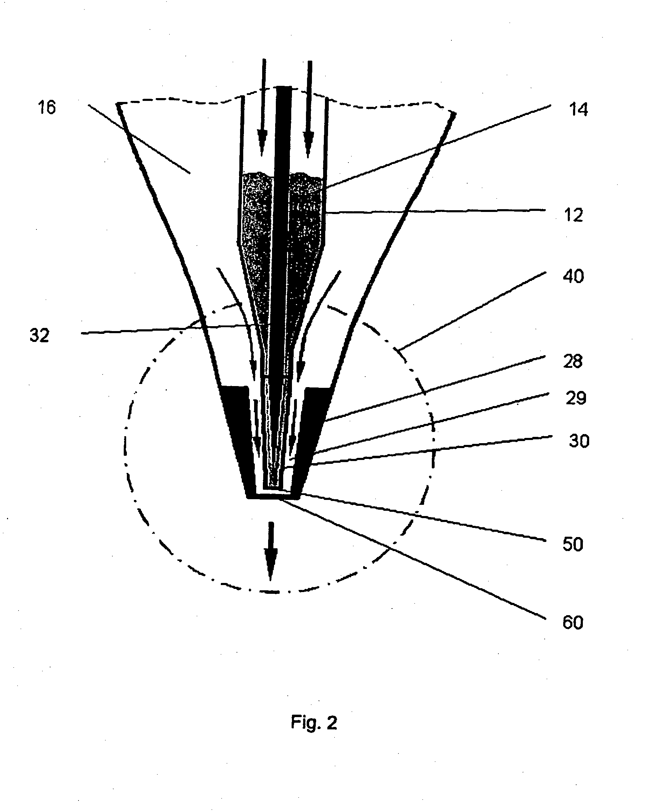

Fig. 2 zeigt das in Fig. 1 schematisch vereinfacht dargestellte Funktionsprinzip der Zerstäubung, wobei der düsennahe Bereich 40 des Applikationssystems 10 zur Verdeutlichung vergrößert dargestellt ist. Dabei geben die Pfeile die Strömungsrichtung des Gases an.

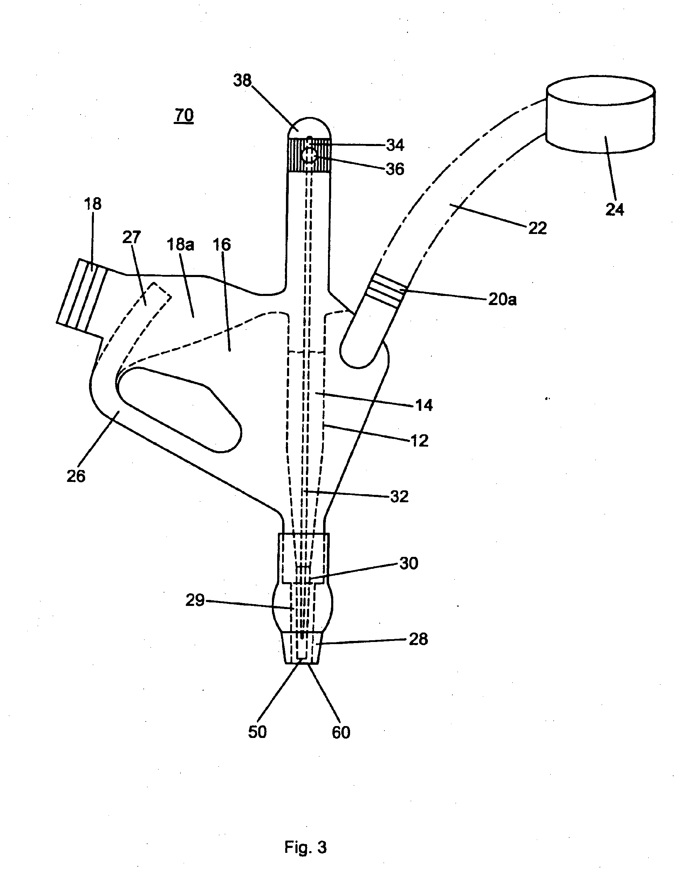

Fig. 3 zeigt ein weiteres Ausführungsbeispiel eines Applikationssystems 70 im Querschnitt. Das Applikationssystem 70 umfasst ein Arzneimittelreservoir 12, das von einem Gasreservoir 16 des Applikationssystems 70 umgeben ist. Das Arzneimittelreservoir 12 ist an seinem Ende, im düsennahen Bereich des Applikationssystems 70, zu einer Kapillare ausgebildet bzw. verjüngt. Je nach zu verabreichender Tagesdosis, befinden sich im Arzneimittelreservoir 12 zwischen 1,5 bis 5 ml eines Arzneimittels 14. Das Arzneimittelreservoir 12 und das Gasreservoir 16 des Applikationssystems 70 sind koaxial ausgebildet und über eine By-passleitung 26 miteinander verbunden. Am oberen Ende des Applikationssystems 70 befinden sich ein Arzneimittelreservoireinlass 18 zur Befüllung eines Arzneimittels 14 und ein Gasreservoireinlass 20 zur Befüllung des Gasreservoirs 16 mit einem Treibgas. Beide Einflüsse können über nichtgezeigte Kappen verschlossen werden.

Der Arzneimittelreservoireinlass 18 ist derart ausgebildet, dass das flüssige Arzneimittel 14 in keinem Fall in den By-pass 26 gelangen und somit aus dem Applikationssystem 70 auslaufen kann. Um dies zu unterbinden wurde das Bypass-Ende 27 derart ausgebildet, dass es weit in die Einlassleitung des Arzneimittelreservoirs 12 hineinragt.

Das Gasreservoir 16 des Applikationssystems 70 ist über eine Schlauchverbindung 22 an einen Gasbehälter 24 angeschlossen. Im düsennahen Bereich ist das Applikationssystem 70 als Rotationskörper, mit sich in Richtung des Düsenaustritts 50 verjüngendem Querschnitt, ausgebildet. Das Arzneistoffreservoir 12 schließt mit seinem verjüngten Ende an die Zerstäubungsdüse 30 an, die innerhalb des Düsenkopfes 28 angeordnet ist. Der Düsenkopf 28 weist entlang seiner Rotationsachse Aussparungen 29 auf, so dass das Gasreservoir 16 mit der Umgebung in Strömungsverbindung steht. Eine in die Zerstäubungsdüse 30 hineinragende Nadel 32, die im oberen Teil des Applikationssystems geführt ist, verengt deren kreisförmigen Querschnitt. Durch Drehung der im Rädelkopf 34 angeordneten Stellschraube (Rändelschraube) ist die Nadel 32 vertikal positionierbar und dadurch die Querschnittsverengung der Zerstäubungsdüse 30 einstellbar.

Im folgenden wird die Funktionsweise eines weiteren, in Fig. 3 dargestellten, Applikationssystems 70 zur Zerstäubung eines zubereiteten Arzneimittels zur perkutanen Arzneimittelverabreichung näher beschrieben.

Je nach Größe der Fläche der zu behandelnden Körperpartie, wird ein genau dosierter Arzneistoff 14, insbesondere in einer Mikroemulsion, vorzugsweise 1,5 bis 5 ml, in das Arzneimittekeservoir 12 über den Arzneimittelreservoireinlass 18 des Applikationssystems 70 eingefüllt.

Zur Zerstäubung des flüssigen Arzneimittels 14 wird das Gasreservoir 16 mit Treibgas , vorzugsweise Sauerstoff, stetig gefüllt, wodurch sich im Gasreservoir 16 ein Überdruck aufbaut. Das Treibgas wird einem Gasbehälter 24 entnommen und unter einem vorbestimmten Druck dem Applikationssystem 70 zugeführt. Hierzu wird das Gasreservoir 16 über eine Schlauchverbindung 22 an einen Gasanschluss 20 des Applikationssystems 70 angeschlossen.

Durch den Überdruck im Gasreservoir 16 wird das Treibgas bis zur Austrittsstelle 50 der Zerstäubungsdüse 30 (Mikrodosierdüse) befördert. Da das Gasreservoir 16 des Applikationssystems 70 im düsennahen Bereich 40 als Rotationskörper, mit sich in Richtung des Düsenaustritts 40 verjüngendem Querschnitt, ausgebildet ist, wird das Treibgas durch den Überdruck im Gasreservoir 16 in Flussrichtung beschleunigt. Der sich infolge der Querschnittsverengung einstellende Staudruck innerhalb des Gasreservoirs 16, wird über eine By-passleitung 26 zur Erzeugung des Vortriebs des flüssigen Arzneimittels 14 auf das Arzneimittelreservoir 12 umgeleitet, wobei der Staudruck das flüssige Arzneimittel durch die Zerstäubungsdüse 30 presst. Hierdurch wird ein gleichmäßiger Vortrieb gewährt.

Das im düsennahen Bereich als innere Kapillare ausgebildete Ende des Arzneimittelreservoirs 12 im Inneren des Gasreservoirs 16 ist derart ausgebildet, dass ein Abriss des Flüssigkeitsstroms 14 unterbunden wird.

Die Aussparungen 29 im Inneren des Düsenkopfes 28 gewährleisten, dass das in Richtung des sich verjüngenden Rotationskörpers 16 beschleunigte Treibgas die Zerstäubungsdüse 30 bis zur Austrittsstelle 50 des Düsenkopfes 28 umströmt.

An der Austrittsstelle 40 der Zerstäubungsdüse 30 angelangt, wird das flüssige Arzneimittel durch den sich in der Austrittsstelle einstellenden Unterdruck (Venturi-Effekt) angesaugt und dabei zerstäubt.

Der Tröpfchengrößendurchmesser bei der Zerstäubung des flüssigen Arzneimittels 14 kann über die Nadel 32 im Inneren der Zerstäubungsdüse 30 variiert werden, indem die Nadel 32 durch Drehung der im Rändelkopf 38 angeordneten Rändelschraube 36 die Nadel 32 fein positioniert wird.

Wird die Zerstäubungsdüse 30 vollkommen durch die Nadel 32 verschlossen, so dass der Massendurchfluss des flüssigen Arzneimittels 14 durch die Zerstäubungsdüse 30 unterbunden ist, so kommt die Zerstäubung des Arzneimittels zum Erliegen. Es fließt sodann lediglich Treibgas durch die Austrittsstelle 60 des Düsenkopfes 28, aufgrund der im Inneren des Düsenkopfes 28 angeordneten Aussparungen 29 entlang der Zerstäubungsdüse 30.

Die Konizität der Nadel 32 ist gegenüber der Konizität der Zerstäubungsdüse 30 zum Zwecke einer breiteren Zerstäubung bzw. eines breiteren Zerstäubungswinkels stärker ausgebildet.

Einen breiteren Zerstäubungswinkel kann man zudem durch die Einbringung eines drallgebenden Mittels in den Düsenkopf 28 verfolgen.

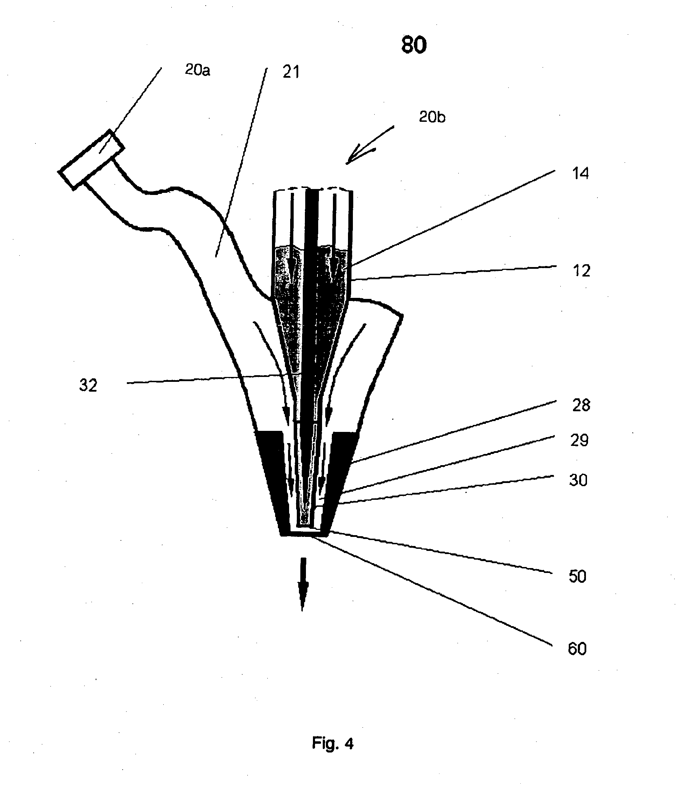

Gemäß einer weiteren Ausführungsform - in Fig. 4 gezeigt - ist das ist das Arzneimittelreservoir an seiner Oberseite direkt mit der Gasquelle verbunden, verfügt somit über einen weiteren Einlass. Dabei kann auf die Venturi-Ausbildung der Düse verzichtet werden, sofern dies zweckmäßig erscheint. Wird jedoch eine Venturi-Zerstäubungsdüse eingesetzt, ist an der Außenseite der Düse eine Gaszuführungseinrichtung entsprechend der Fig. 2 vorgesehen. Bis auf den Düsenbereich kann also auf ein Gasreservoir verzichtet werden, sofern nur eine Gaszuführung in den Düsenbereich vorgesehen ist, wie dies in Fig. 4 dargestellt ist.

Im Folgenden werden anhand von Beispielen Mikroemulsionen und deren Herstellung beschrieben, die im Rahmen dieser Erfindung mit Sauerstoff angereichert werden können, beispielsweise bei der Verabreichung im erfindungsgemäßen Applikationssystem. Diese Beispiele sollen nicht einschränkend wirken.

### Beispiel 1: Herstellung einer Wasser-in-Öl-Mikroemulsion (I)

5g Tween^{®} 80 Werden mit 10 g Span^{®} 20 und 5 g Ethanol gemischt und es werden 75 g Isopropylmyristat zugesetzt. Zu dieser Mischung werden unter Rühren tropfenweise 5 g Wasser zugegeben. Dies ergibt 100 g einer Wasser-in-Öl-Mikroemulsion (I).

### Beispiel 2: Herstellung einer Wasser-in-Öl-Mikroemulsion (II)

14 g Spann^{®} 20 werden mit 21 g Synperonic^{®} PEL 101 gemischt. Dazu werden 60 g Isopropylpalmiat zugesetzt. Zu dieser Mischung werden unter Rühren tropfenweise 5 g Wasser zugegeben. Dies ergibt 100 g einer Wasser-in-Öl-Mikroemulsion (II).

### Beispiel 3: Herstellung einer Öl-in-Wasser-Mikroemulsion (III)

4g Tween^{®} 80 werden mit 12 g Synperonic^{®} PEL 101 gemischt: Dazu werden 5 g Isopropylmyristat zugesetzt. Zu dieser Mischung werden unter Rühren 79 g eines Gemischs Wasser / Polypropylenglycol (1:2) (Gewichtsverhältnis) zugegeben. Dies ergibt 100 g einer Öl-in-Wasser-Mikroemulsion (III).

### Beispiel 4: Zubereitung eines Arzneimittels mit dem Arzneistoff Procain zur lokalen Schmerzbekämpfung auf Basis einer Öl-in-Wasser-Mikroemulsion:

2 g Procain-Chlorid werden in 5 ml Wasser gelöst. Die Lösung wird unter Rühren in 93 g der Mikroemulsion III gegeben. Dies ergibt 100g des Arzneimittels.

### Beispiel 5: Zubereitung eines weiteren Arzneimittels mit dem Arzneistoff Procain zur lokalen Schmerzbekämpfung auf Basis einer Wasser-in-Öl-Mikroemulsion:

2 g Procain-Chlorid werden in 5 g 0,01 M NaOH gelöst. Die Lösung wird unter Rühren tropfenweise in 93 g der Mikroemulsion I gegeben. Dies ergibt 100g des Arzneimittels.

### Beispiel 6: Zubereitung eines Arzneimittels mit dem Arzneistoff Lidocain zur lokalen Schmerzbekämpfung auf Basis einer Wasser-in-Öl-Mikroemulsion:

2 g Lidocain werden in 98 ml der Mikroemulsion II gelöst. Dies ergibt 100 g des Arzneimittels.

### Beispiel 7: Zubereitung eines Arzneimittels mit dem Arzneistoff Diclofenac zur lokalen Bekämpfung schmerzhafter Entzündungen auf Basis einer Wasser-in-Öl-Mikroemulsion:

2 g Lidocain, 2 g Diclofenac und 0,05 g Capsaicin werden nacheinander in 95,95 g der Mikroemulsion (II) gelöst. Dies ergibt 100g des Arzneimittels.

## Patentansprüche

1. Eine nach Zerstäuben mit einem Treibgas mit Sauerstoff angereichert vorliegende Mikroemulsion, enthaltend mindestens einen Arzneistoff zur perkutanen Verabreichung, **dadurch gekennzeichnet, dass** die Sauerstoffanreicherung durch Zerstäuben mit einem sauerstoffhaltigen Treibgas oder durch Zerstäuben mit einem Treibgas einer Mikroemulsion, enthaltend einen Zusatz zur Verbesserung der Sauerstoffversorgung, vorliegt.

2. Mikroemulsion gemäß Anspruch 1, **dadurch gekennzeichnet, dass** das Treibgas ausgewählt ist aus Inertgas, Luft oder einem Treibgas mit einer Sauerstoff-haltigen Atmosphäre größer 25 Vol..% des Treibgases..

3. Mikroemulsion nach Anspruch 1 oder 2, **dadurch gekennzeichnet, dass** ein Zusatz zur Verbesserung der Sauerstoffversorgung der Haut, ausgewählt aus Hämoglobin oder Myoglobin, enthalten ist.

4. Mikroemulsion nach einem der Ansprüche 1 bis 3, **dadurch gekennzeichnet, dass** der Arzneistoff ausgewählt ist aus der Gruppe, umfassend analgetisch, anästhesierend, antiphlogistisch, spasmolytisch, hyperämisierend wirkende Arzneistoffe.

5. Mikroemulsion nach einem der Ansprüche 1 bis 3, **dadurch gekennzeichnet, dass** der Arzneistoff ausgewählt ist aus der Gruppe, umfassend antibakteriell, antikoagulierend, aquaretisch, antiviral, antioxidativ, epithelisierend, keratolytisch, hautnährend, wundheilend, antimykotisch wirkende Arzneistoffe.

6. Mikroemulsion nach einem der Ansprüche 1 bis 3, **dadurch gekennzeichnet, dass** der Arzneistoff ausgewählt ist aus der Gruppe, umfassend antiallergisch, ödemprotektiv, antimykotisch, hautregenerierend, brennlindernd, hämolytisch, mitosehemmend, bindegewebsregenerierend, antispastisch wirkenden Arzneistoffen.

7. Mikroemulsion nach einem der Ansprüche 1 bis 3, **dadurch gekennzeichnet, dass** der Arzneistoff ausgewählt ist aus der Gruppe, umfassend antimikrobiell, immunmodulatorisch, juckreizstillend, adstringierend, lokal-anästhesierend wirkende Arzneimittel.

8. Mikroemulsion nach einem der Ansprüche 1 bis 7, **dadurch gekennzeichnet, dass** Kombinationen aus Arzneistoffen vorliegen.

9. Applikationssystem zur perkutanen Verabreichung von Arzneimitteln auf Basis von Mikroemulsionen, **dadurch gekennzeichnet, dass** das System einen Behälter für eine Mikroemulsion sowie eine daran angeschlossene Zerstäubungseinheit mit Verbindung zu einer unter Druck stehenden (Treib)Gasquelle aufweist.

10. Applikationssystem gemäß Anspruch 9, **gekennzeichnet durch**:
a) ein Arzneimittelreservoir (12), welches das flüssige Arzneimittel (14), in Form der Mikroemulsion, enthält, b) einen ersten Gasanschluss (18), an dem Gas unter einem vorbestimmten Druck über eine Gaszuführung (22) in das Arzneimittelreservoir (12) zuführbar ist, c) einen Arzneimittelreservoireinlass (18), an dem das flüssige Arzneimittel (14) zuführbar ist, d) einen Düsenkopf (28) mit Aussparungen (29), der am Ende des Arzneimittelreservoirs (12) angeordnet ist, e) eine Zerstäubungsdüse (30), die im Düsenkopf (28). angeordnet ist und mit dem Arzneimittelreservoir (12) in Strömungsverbindung steht, und an der ein Spektrum von Tröpfchengrößen generierbar ist, wobei der Düsenkopf (28) und die Zerstäubungsdüse (30) eine Venturi- Anordnung bilden, und der Düsenkopf (28) einen Ringraum um die Zerstäubungsdüse (30) aufweist, und f) einen zweiten Gasanschluss (20), der im Bereich des Düsenkopfes (28) angeordnet und über eine Gaszuführung (21) mit dem Ringraum und den Aussparungen (29) in Strömungsverbindung steht, wobei die an der Austrittsstelle der Zerstäubungsdüse (30) mittels Druck generierten Tröpfchen durch den Venruri-Effekt zerstäubt werden.

11. Applikationssystem nach einem der vorhergehenden Ansprüche 9 oder 10, **dadurch gekennzeichnet, dass** das Gasreservoir mit dem Arzneimittelreservoir (12) über eine By-Passleitung (26), verbunden ist.

12. Applikationssystem nach einem der vorhergehenden Ansprüche 9 bis 11, **dadurch gekennzeichnet, dass** das unter Druck stehende Gas Sauerstoff, Luft, Stickstoff oder ein Edelgas (Helium, Argon), vorzugsweise Sauerstoff, ist.

13. System nach einem der Ansprüche 9 bis 12, **dadurch gekennzeichnet, dass** eine Mikroemulsion mit einem Arzneimittel gemäß einem der Ansprüche 1 bis 8 enthalten ist.

14. Verwendung einer Mikroemulsion gemäß einem der Ansprüche 1 bis 8 zur Herstellung eines zerstäubbaren Mittels zur Behandlung rheumatischer Beschwerden, des peripheren, komplexen Schmerzsyndroms, von Wunden, Prellungen, Zerrungen, Sportverletzungen, Neuralgien, viralen Infektionen, Hämatomen oder degenerierter Haut.

## Claims

1. A microemulsion assuming an oxygenated form after atomisation with a propellant gas and containing at least one pharmaceutical substance for percutaneous administration, **characterised in that** oxygenation proceeds by atomisation with an oxygen-containing propellant gas or by atomisation with a propellant gas of a microemulsion containing an additive for improving oxygen supply.

2. A microemulsion according to claim 1, **characterised in that** the propellant gas is selected from inert gas, air or a propellant gas with an oxygen-containing atmosphere of greater than 25 vol.% of the propellant gas.

3. A microemulsion according to claim 1 or claim 2, **characterised in that** it contains an additive for improving oxygen supply to the skin, selected from haemoglobin or myoglobin.

4. A microemulsion according to any one of claims 1 to 3, **characterised in that** the pharmaceutical substance is selected from the group comprising pharmaceutical substances having an analgesic, anaesthetic, antiinflammatory, spasmolytic or circulation-boosting action.

5. A microemulsion according to any one of claims 1 to 3, **characterised in that** the pharmaceutical substance is selected from the group comprising pharmaceutical substances having an antibacterial, anticoagulant, diuretic, antiviral, antioxidant, epithelialising, keratolytic, skin-nourishing, wound-healing or antimycotic action.

6. A microemulsion according to any one of claims 1 to 3, **characterised in that** the pharmaceutical substance is selected from the group comprising pharmaceutical substances having an antiallergic, oedema-protective, antimycotic, skin-regenerative, burn-alleviating, haemolytic, antimitotic, connective tissue-regenerative or antispasmodic action.

7. A microemulsion according to any one of claims 1 to 3, **characterised in that** the pharmaceutical substance is selected from the group comprising medicaments having an antimicrobial, immunomodulatory, itch-relieving, astringent or local anaesthetic action.

8. A microemulsion according to any one of claims 1 to 7, **characterised in that** combinations of pharmaceutical substances are present.

9. An administration system for percutaneous administration of medicaments based on microemulsions, **characterised in that** the system comprises a container for a microemulsion and, connected thereto, an atomising unit with a connection to a pressurised (propellant) gas source.

10. An administration system according to claim 9, **characterised by**:
a) a medicament reservoir (12) which contains the liquid medicament (14) in the form of the microemulsion, b) a first gas port (18) to which gas under a predetermined pressure can be supplied into the medicament reservoir (12) via a gas supply (22), c) a medicament reservoir inlet (18) to which the liquid medicament (14) can be supplied, d) a nozzle head (28) with recesses (29) which is arranged on the end of the medicament reservoir (12), e) an atomising nozzle (30) which is arranged in the nozzle head (28) and is in fluidic connection with the medicament reservoir (12), and at which a range of droplet sizes can be generated, wherein the nozzle head (28) and atomising nozzle (30) form a Venturi arrangement, and the nozzle head (28) has an annular space around the atomising nozzle (30), and f) a second gas port (20) which is arranged in the region of the nozzle head (28) and is in fluidic connection via a gas supply (21) with the annular space and the recesses (29), wherein the droplets generated by pressure at the point of emergence from the atomising nozzle (30) are atomised by the Venturi effect.

11. An administration system according to either one of the preceding claims 9 or 10, **characterised in that** the gas reservoir is connected to the medicament reservoir (12) via a bypass line (26).

12. An administration system according to any one of the preceding claims 9 to 11, **characterised in that** the pressurised gas is oxygen, air, nitrogen or a noble gas (helium, argon), preferably oxygen.

13. A system according to any one of claims 9 to 12, **characterised in that** it contains a microemulsion with a medicament according to any one of claims 1 to 8.

14. Use of a microemulsion according to any one of claims 1 to 8 for producing an atomisable agent for treating rheumatic diseases, complex regional pain syndrome, wounds, contusions, strains, sports injuries, neuralgia, viral infections, haematomas or degenerated skin.

## Revendications

1. Microémulsion disponible après une pulvérisation à l'aide d'un gaz propulseur enrichi en oxygène, contenant au moins une substance médicamenteuse pour une administration percutanée, **caractérisée en ce que** l'enrichissement en oxygène est disponible par le biais d'une pulvérisation à l'aide d'un gaz propulseur contenant de l'oxygène ou d'une pulvérisation d'une microémulsion à l'aide d'un gaz propulseur, contenant un additif pour améliorer l'apport d'oxygène.

2. Microémulsion selon la revendication 1, **caractérisée en ce que** le gaz propulseur est sélectionné parmi un gaz inerte, l'air ou un gaz propulseur ayant une atmosphère à teneur en oxygène supérieure à 25 % en volume du gaz propulseur.

3. Microémulsion selon la revendication 1 ou 2, **caractérisée en ce qu'**un additif pour améliorer l'apport d'oxygène à la peau, sélectionné parmi l'hémoglobine ou la myoglobine, est contenu.

4. Microémulsion selon l'une des revendications 1 à 3, **caractérisée en ce que** la substance médicamenteuse est sélectionnée dans le groupe comprenant les substances médicamenteuses à action analgésique, anesthésiante, antiphlogistique, spasmolytique, hyperémiante.

5. Microémulsion selon l'une des revendications 1 à 3, **caractérisée en ce que** la substance médicamenteuse est sélectionnée dans le groupe comprenant les substances médicamenteuses à action antibactérienne, anticoagulante, aquarétique, antivirale, antioxydante, épithélialisante, kératolytique, nourrissante pour la peau, cicatrisante, antimycosique.

6. Microémulsion selon l'une des revendications 1 à 3, **caractérisée en ce que** la substance médicamenteuse est sélectionnée dans le groupe comprenant les substances médicamenteuses à action antiallergique, protectrice contre les oedèmes, antimycosique, régénératrice pour la peau, soulageante pour les brûlures, hémolytique, inhibitrice de la mitose, régénératrice du tissu conjonctif, antispastique.

7. Microémulsion selon l'une des revendications 1 à 3, **caractérisée en ce que** la substance médicamenteuse est sélectionnée dans le groupe comprenant les médicaments à action antimicrobienne, immunomodulatrice, apaisante pour les démangeaisons, astringente, anesthésiante localement.

8. Microémulsion selon l'une des revendications 1 à 7, **caractérisée en ce que** des combinaisons de substances médicamenteuses sont présentes.

9. Système d'application pour une administration percutanée de médicaments à base de microémulsions, **caractérisé en ce que** le système présente un récipient pour une microémulsion ainsi qu'une unité de pulvérisation raccordée à celui-ci avec une connexion à une source de gaz (propulseur) sous pression.

10. Système d'application selon la revendication 9, **caractérisé par** :
a) un réservoir de médicament (12), qui contient le médicament liquide (14), sous la forme d'une microémulsion, b) un premier raccord de gaz (18), au niveau duquel un gaz peut être amené sous une pression prédéterminée par le biais d'une amenée de gaz (22) dans le réservoir de médicament (12), c) une entrée de réservoir de médicament (18), au niveau de laquelle le médicament liquide (14) peut être amené, d) une tête de buse (28) comportant des évidements (29), qui est disposée à l'extrémité du réservoir de médicament (12), e) une buse de pulvérisation (30), qui est disposée dans la tête de buse (28) et qui est en communication d'écoulement avec le réservoir de médicament (12), et au niveau de laquelle un spectre de dimensions de gouttelettes peut être généré, la tête de buse (28) et la buse de pulvérisation (30) formant un venturi, et la tête de buse (28) présentant un espace annulaire autour de la buse de pulvérisation (30), et f) un deuxième raccord de gaz (20), qui est disposé dans la zone de la tête de buse (28) et qui est en communication d'écoulement avec l'espace annulaire et les évidements (29) par le biais d'une amenée de gaz (21), les gouttelettes générées par pression au niveau du point de sortie de la buse de pulvérisation (30) étant pulvérisées par le biais de l'effet venturi.

11. Système d'application selon l'une des revendications précédentes 9 ou 10, **caractérisé en ce que** le réservoir de gaz est relié au réservoir de médicament (12) par le biais d'une conduite de dérivation (26).

12. Système d'application selon l'une des revendications précédentes 9 à 11, **caractérisé en ce que** le gaz sous pression est l'oxygène, l'air, l'azote ou un gaz rare (hélium, argon), de préférence l'oxygène.

13. Système selon l'une des revendications 9 à 12, **caractérisé en ce qu'**une microémulsion est contenue avec un médicament selon l'une des revendications 1 à 8.

14. Utilisation d'une microémulsion selon l'une des revendications 1 à 8 pour la fabrication d'un agent apte à être pulvérisé pour le traitement des douleurs rhumatismales, du syndrome de la douleur périphérique complexe, des plaies, des contusions, des élongations, des blessures sportives, des névralgies, des infections virales, des hématomes ou de la dégénérescence de la peau.
